(19) Europäisches Patentamt
European Patent Office
Office européen des brevets

(11) EP 2 978 427 B1

(12) EUROPÄISCHE PATENTSCHRIFT

(45) Veröffentlichungstag und Bekanntmachung des Hinweises auf die Patenterteilung:
**08.08.2018 Patentblatt 2018/32**

(21) Anmeldenummer: **14713115.5**

(22) Anmeldetag: **27.03.2014**

(51) Int Cl.:
***A61K 31/4412*** (2006.01) ***A61P 7/06*** (2006.01)
***A61K 33/26*** (2006.01)

(86) Internationale Anmeldenummer:
**PCT/EP2014/056146**

(87) Internationale Veröffentlichungsnummer:
**WO 2014/154797 (02.10.2014 Gazette 2014/40)**

# (54) FE(III)-KOMPLEXVERBINDUNGEN ZUR BEHANDLUNG UND PROPHYLAXE VON EISENMANGELERSCHEINUNGEN UND EISENMANGELANÄMIEN

FE(III) COMPLEX COMPOUNDS FOR TREATING AND PROPHYLAXIS FOR ANAEMIA SYMPTOMS AND ANAEMIA

LIAISONS DE COMPLEXES DE FE(III) POUR LE TRAITEMENT ET LA PROPHYLAXIE DES CARENCES EN FER ET DES ANÉMIES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.03.2013 EP 13161698**

(43) Veröffentlichungstag der Anmeldung:
**03.02.2016 Patentblatt 2016/05**

(73) Patentinhaber: **Vifor (International) AG**
**9001 St. Gallen (CH)**

(72) Erfinder:
- **BARK, Thomas**
  **CH-8050 Zürich (CH)**
- **BUHR, Wilm**
  **78465 Konstanz (DE)**
- **BURCKHARDT, Susanna**
  **CH-8049 Zürich (CH)**
- **BURGERT, Michael**
  **88045 Friedrichshafen (DE)**
- **CANCLINI, Camillo**
  **CH-9000 St. Gallen (CH)**
- **DÜRRENBERGER, Franz**
  **CH-4143 Dornach (CH)**
- **FUNK, Felix**
  **CH-8404 Winterthur (CH)**
- **GEISSER, Peter Otto**
  **CH-9014 St. Gallen (CH)**
- **KALOGERAKIS, Aris**
  **CH-8400 Winterthur (CH)**
- **MAYER, Simona**
  **CH-9055 Bühler (CH)**
- **PHILIPP, Erik**
  **CH-9320 Arbon (CH)**
- **SIEBER, Diana**
  **CH-9030 Abtwil (CH)**
- **SCHMITT, Jörg**
  **78343 Gaienhofen (DE)**
- **SCHWARZ, Katrin**
  **CH-9000 St. Gallen (CH)**
- **REIM, Stefan**
  **CH-8404 Stadel Winterthur (CH)**

(74) Vertreter: **Gille Hrabal**
**Brucknerstrasse 20**
**40593 Düsseldorf (DE)**

(56) Entgegenhaltungen:
**WO-A1-2011/117225 WO-A1-2012/163938**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 978 427 B1

## Beschreibung

### Einleintung:

Die Erfindung betrifft Eisen(III)-3-hydroxy-isonicotinamid-Komplexverbindungen und deren sie umfassende pharmazeutischen Zusammensetzungen zur Verwendung als Arzneimittel, insbesondere zur Behandlung und/oder zur Prophylaxe von Eisenmangelerscheinungen und Eisenmangelanämien.

### Hintergrund:

Eisen ist ein essentielles Spurenelement für fast alle Lebewesen und ist dabei insbesondere für das Wachstum und die Blutbildung relevant. Die Balance des Eisenhaushaltes wird dabei primär auf der Ebene der Eisenwiedergewinnung aus Hämoglobin von alternden Erythrozyten und der duodenalen Absorption von nahrungsgebundenem Eisen reguliert. Das freigesetzte Eisen wird über den Darm insbesondere durch spezifische Transportsysteme (DMT-1, Ferroportin, Transferrin, Transferrin Rezeptoren) aufgenommen, in den Blutkreislauf transportiert und hierüber in die entsprechenden Gewebe und Organe weitergeleitet.

Das Element Eisen ist im menschlichen Körper unter anderem für den Sauerstofftransport, die Sauerstoffaufnahme, Zellfunktionen, wie den mitochondrialen Elektronentransport und letztlich für den gesamten Energiestoffwechsel von großer Bedeutung.

Der Körper eines Menschen enthält im Durchschnitt 4 bis 5 g Eisen, wobei dies in Enzymen, in Hämoglobin und Myoglobin, sowie als Depot- oder Reserve-Eisen in Form von Ferritin und Hämosiderin vorliegt.

Etwa die Hälfte dieses Eisens ca. 2 g liegt als Häm Eisen gebunden im Hämoglobin der roten Blutkörperchen vor. Da Erythrozyten nur eine begrenzte Lebensdauer aufweisen (75-150 Tage), müssen ständig neue gebildet und alte eliminiert werden (über 2 Mio. Erythrozyten werden pro Sekunde neu gebildet). Diese hohe Neubildungskapazität wird durch Makrophagen erreicht, indem diese die alternden Erythrozyten phagozytotisch aufnehmen, lysieren und so das enthaltene Eisen für den Eisenhaushalt rezyklieren können. Somit wird die täglich für die Erythropoese benötige Eisenmenge von ca. 25 mg grösstenteils bereit gestellt.

Der tägliche Eisenbedarf eines erwachsenen Menschen beträgt zwischen 0,5 und 1,5 mg pro Tag, bei Kleinkindern sowie bei Frauen in der Schwangerschaft liegt der Eisenbedarf bei 2 bis 5 mg pro Tag. Die täglichen Eisenverluste, z.B. durch Abschilferung von Haut- und Epithelzellen ist gering, erhöhte Eisenverluste treten beispielsweise bei der Menstruationsblutung bei Frauen auf. Generell können Blutverluste den Eisenhaushalt beträchtlich verringern, da pro 2 ml Blut etwa 1 mg Eisen verloren gehen. Der normale tägliche Eisenverlust von ca. 1 mg wird üblicherweise bei einem erwachsenen, gesunden Menschen über die tägliche Nahrungsaufnahme wieder ersetzt. Der Eisenhaushalt wird über Resorption reguliert, wobei die Resorptionsquote des in der Nahrung vorhandenen Eisens zwischen 6 und 12 % beträgt, bei Eisenmangel beträgt die Resorptionsquote bis zu 25 %. Die Resorptionsquote wird vom Organismus in Abhängigkeit vom Eisenbedarf und der Eisenspeichergröße reguliert. Dabei nutzt der menschliche Organismus sowohl zweiwertige als auch dreiwertige Eisenionen. Üblicherweise werden Eisen(III)-Verbindungen bei ausreichend saurem pH-Wert im Magen gelöst und damit für die Resorption verfügbar gemacht. Die Resorption des Eisens findet im oberen Dünndarm durch Mukosazellen statt. Dabei wird dreiwertiges nicht Häm Eisen für die Resorption z.B. durch Ferrireduktase (membranständiges, duodenales Cytochrom b) in der Darmzellmembran zunächst zu Fe(II) reduziert, um dann durch das Transportprotein DMT1 (divalent metal transporter 1) in die Darmzellen transportiert werden zu können. Dagegen gelangt Häm Eisen unverändert über die Zellmembran in die Enterozyten. In den Enterozyten wird Eisen entweder als Depot-Eisen in Ferritin gespeichert oder durch das Transportprotein Ferroportin ins Blut abgegeben. In diesem Vorgang spielt Hepcidin eine zentrale Rolle, da es den wesentlichen Regulationsfaktor der Eisenaufnahme darstellt. Das durch das Ferroportin ins Blut transportierte zweiwertige Eisen wird durch Oxidasen (Ceruloplasmin, Hephaestin) in dreiwertiges Eisen überführt, welches dann mittels Transferrin an die relevanten Stellen im Organismus transportiert wird (s. zum Beispiel: "Balancing acts: molecular control of mammalian iron metabolism". M.W. Hentze, Cell 117, 2004, 285-297.)

Der Organismus von Säugetieren kann Eisen nicht aktiv ausscheiden. Der Eisenmetabolismus wird im Wesentlichen über die zelluläre Freisetzung von Eisen aus Makrophagen, Hepatocyten und Enterozyten durch das Hepcidin gesteuert.

Ein verringerter Serum-Eisenspiegel führt in krankhaften Fällen zu einem verringerten Gehalt an Hämoglobin, verringerter Erythrozytenproduktion und damit zu einer Anämie.

Äussere Symptome von Anämien beinhalten Müdigkeit, Blässe sowie verringerte Aufmerksamkeitskapazitäten. Die klinischen Symptome einer Anämie beinhalten niedrige Serum-Eisengehalte (Hypoferrämie), geringe Hämoglobingehalte, geringe Hämatokritlevel sowie eine reduzierte Anzahl an roten Blutkörperchen, reduzierte Retikulozyten und erhöhte Werte an löslichen Transferrinrezeptoren.

Eisenmangelerscheinungen oder Eisenanämien werden durch Eisenzufuhr behandelt. Dabei erfolgt die Substitution mit Eisen entweder auf dem oralen Weg oder durch intravenöse Eisengabe. Außerdem können zur Ankurbelung

der Bildung von roten Blutkörperchen auch Erythropoetin und andere Erythropoese-stimulierende Substanzen bei der Behandlung von Anämien eingesetzt werden.

Anämie kann oft auf Mangelernährung bzw. eisenarme Diäten oder unausgewogene, eisenarme Ernährungsgewohnheiten zurück geführt werden. Außerdem treten Anämien durch verringerte bzw. schlechte Eisenabsorption beispielsweise aufgrund von Gastrektomien oder von Erkrankungen wie Crohn's-Disease auf. Auch kann ein Eisenmangel infolge eines erhöhten Blutverlustes z.B. durch eine Verletzung, starke Menstruationsblutung oder Blutspende auftreten. Auch ist ein erhöhter Eisenbedarf in der Wachstumsphase Heranwachsender und Kinder sowie von Schwangeren bekannt. Da ein Eisenmangel nicht nur zu einer verringerten Bildung von roten Blutkörperchen, sondern damit auch zu einer schlechten Versorgung des Organismus mit Sauerstoff führt, was zu den oben genannten Symptomen wie Müdigkeit, Blässe und Konzentrationsschwäche auch gerade bei Heranwachsenden zu langfristigen negativen Auswirkungen auf die kognitive Entwicklung führen kann, ist eine gut wirksame und gut verträgliche Therapie von besonderem Interesse.

Durch die Verwendung der erfindungsgemäßen Fe(III)-Komplexverbindungen besteht die Möglichkeit, Eisenmangelerscheinungen und Eisenmangelanämien durch orale Applikation effektiv zu behandeln, ohne das grosse Nebenwirkungspotential der klassischen Präparate, der Fe(II)-Eisensalze, wie $FeSO_4$, hervorgerufen durch Oxidativen Stress, in Kauf zu nehmen. Somit wird eine schlechte Compliance vermieden, die oft Grund für die mangelnde Beseitigung des Eisenmangelzustands darstellt.

**Stand der Technik:**

Aus dem Stand der Technik ist eine Vielzahl von Eisenkomplexen für die Behandlung von Eisenmangelzuständen bekannt.

Ein sehr grosser Anteil dieser Komplex-Verbindungen besteht aus polymeren Strukturen. Hierbei handelt es sich bei den meisten Komplex-Verbindungen um Eisen-Polysaccharid-Komplexverbindungen (WO20081455586, WO2007062546, WO20040437865, US2003236224, EP150085). Gerade aus diesem Bereich sind schon Medikamente auf dem Markt verfügbar (wie Maltofer, Venofer, Ferinject, Dexferrum, Ferumoxytol).

Ein anderer grosser Bestandteil der Gruppe der polymeren Komplexverbindungen sind die Eisen-Peptid-Komplexverbindungen (CN101481404, EP939083, JP02083400).

In der Literatur sind auch Fe-Komplexverbindungen beschrieben, die sich von Makromolekülen, wie Hämoglobin, Chlorophyll, Curcumin und Heparin strukturell herleiten (US474670, CN1687089, Biometals, 2009, 22, 701-710).

Ferner sind in der Literatur auch niedermolekulare Fe-Komplexverbindungen beschrieben. Eine Vielzahl dieser Fe-Komplexverbindungen umfasst Carbonsäuren und Aminosäuren als Liganden. Besonders stehen hier Aspartat (US2009035385) und Citrat (EP308362) als Liganden im Vordergrund. In diesem Zusammenhang werden auch Fe-Komplexverbindungen, die derivatisierte Phenylalanin-Reste als Liganden beinhalten beschrieben (ES2044777).

Im weiteren sind in der Literatur Fe-Komplexverbindungen beschrieben, die aus monomeren Zuckereinheiten oder aus einer Kombination von monomeren und polymeren Einheiten aufgebaut sind (FR19671016).

In der Literatur sind auch Hydroxypyron- und Hydroxypyridon-Fe-Komplexverbindungen beschrieben (EP159194, EP138420, EP107458, EP0120670). Analog hierzu sind auch die entsprechenden 5-Ringsysteme, die Hydroxyfuranon-Fe-Komplexverbindungen beschrieben (WO2006037449). Insbesondere die Hydroxypyridon-Fe-Komplex-verbindungen weisen jedoch eine vergleichsweise geringe Wasserlöslichkeit auf, weshalb sie insbesondere für die orale Verabreichung weniger geeignet sind. Desweiteren weisen die Hydroxypyridon-Fe-Komplexverbindungen eine vergleichsweise geringe Eisenutilisation auf.

Ausserdem sind in der Literatur auch Fe-Komplexverbindungen mit Pyrimidin-2-ol-1-oxid Liganden beschrieben, die für die Behandlung von Eisenmangelanämien eingesetzt werden sollen (WO2012130882). Die WO2012163938 beschreibt Eisen(III)-2,4-dioxo-1-carbonyl-Komplexverbindungen mit Liganden der Formel (I):

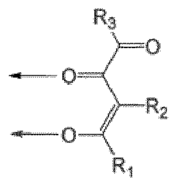

(I)

die ebenfalls für die Behandlung von Eisenmangelanämien eingesetzt werden sollen. Es ist ersichtlich, dass durch diese Ligandenstruktur die Liganden gemäß der allgemeinen Formel (I) der vorliegenden Anmeldung

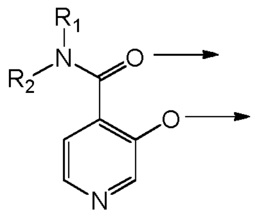

(I)

nicht verwirklicht wird, selbst wenn die darin mit $R_1$ und $R_2$ bezeichneten Reste gemeinsam einen geeigneten heterocyclischen aromatischen Ring bilden würden. Der verbleibende Carbonylrest ($R_3$-C(=O)-) ist eindeutig unterschiedlich von der in der vorliegenden Anmeldung an entsprechender Position zwingend vorhandenen AmidGruppe.

Im weiteren sind in der Literatur auch Fe-Komplexe mit β-Ketoamid-Liganden beschrieben, deren Verwendung für die Behandlung von Eisenmangelzuständen vorgeschlagen werden. So offenbart die WO2011117225 Eisen-Komplexverbindungen, die aus β-Ketoamid-Liganden

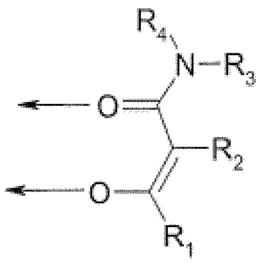

(I)

gebildet werden. Bezüglich der Bedeutung der hierin definierten Substituenten $R_1$ und $R_2$ wird erwähnt, daß diese auch gemeinsam mit den Kohlenstoffatomen, an die sie gebunden sind, einen gegebenenfalls substituierten 5- oder 6-gliedrigen Ring bilden können, der gegebenenfalls ein oder mehrere Heteroatome aufweisen kann. Allerdings wird keine Ringbildung in Form aromatischer Heterocyclen erwähnt. Als konkrete Beispiele für Liganden mit einer solchen Ringbildung durch $R_1$ und $R_2$ zeigt die WO2011117225

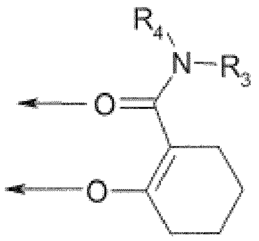

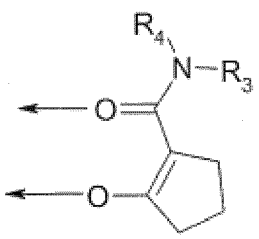

Die einzige konkret offenbarte Eisen(III)-Komplex-Beispielverbindung mit einer solchen Ringbildung durch die Substituenten $R_1$ und $R_2$ zeigt Beispiel 66 mit einem Tris-(N,N-Dimethyl-2-oxocyclopentan:

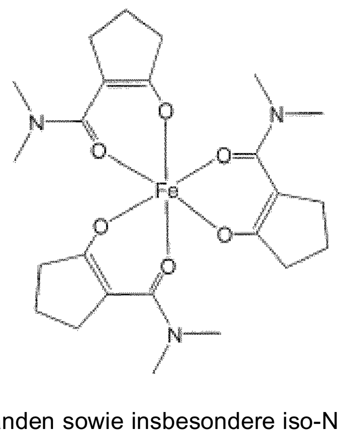

Heterocyclische aromatische Liganden sowie insbesondere iso-Nicotinamid-Liganden oder daraus gebildete

Eisen(III)-Komplexe erwähnt die WO2011117225 nicht. Die darin gezeigten Cyclopentan- oder Cyclohexan-carboxamid-Eisen(III)-Komplexe weisen jedoch eine so geringe Stabilität auf, daß diese so gut wie nicht in pharmazeutischen Zusammensetzungen formulierbar sind und damit für eine medizinische Anwendung ungeeignet sind.

Einen anderen wichtigen Bestandteil für die Behandlung von Eisenmangelerscheinungen und Eisenmangelanämien stellen die Eisensalze (z.B. Eisen(II)sulfat, Eisen(II)fumarat, Eisen(III)chlorid, Eisen(II)aspartat, Eisen(II)succinat) dar.

Ein grosses Problem dieser Eisensalze stellt ihre teilweise hohe Unverträglichkeit (bis zu 50%) in Form von Übelkeit, Erbrechen, Durchfall aber auch Obstipation und Krämpfe dar. Ausserdem kommt es bei der Verwendung von diesen Eisen(II)-salzen zum Auftreten freier Fe(II)Ionen, die die Bildung (u.a. Fenton Reaktion) von reaktiven Sauerstoff Spezies (ROS) katalysieren. Durch diese ROS kommt es zur Beschädigung von DNA, Lipiden, Proteinen und Kohlenhydraten, was weitreichende Auswirkungen auf Zellen, Gewebe und Organe hat. Diese Problematik ist bekannt und wird in der Literatur weitgehend als Ursache für die hohe Unverträglichkeit angesehen und als Oxidativer Stress bezeichnet.

**Aufgabenstellung:**

Die Aufgabe der vorliegenden Erfindung bestand darin, neue therapeutisch wirksame Verbindungen mit guter Aktivität bzw. Eisenutilisation, Komplexstabilität und Löslichkeit, insbesondere guter Stabilität und guter Löslichkeit im pH-Wert neutraler wässriger Medien zu entwickeln, die für eine oralen Behandlung von Eisenmangelerscheinungen und Eisenmangelanämien eine effektive Therapie darstellen. Gerade eine gute Stabilität und eine gute Löslichkeit sind sehr wichtig für eine effektive orale Eisentherapie.

Ausserdem sollten diese Eisenkomplexe gleichzeitig deutlich weniger Nebenwirkungen bzw. eine geringe Toxizität aufzeigen, insbesondere im Vergleich zu den klassisch verwendeten Fe(II)-Salzen. Im Vergleich zu den bekannten polymeren Eisenkomplexverbindungen sollten diese neuen Eisenkomplexe eine definierte Struktur (Stöchiometrie) aufweisen und durch einfache Synthesewege darstellbar sein. Diese Ziele wurden durch die Entwicklung neuartiger Fe(III)-Komplexverbindungen erreicht.

Weiterhin sollten die neuen Eisenkomplexe so beschaffen sein, das sie direkt über die Membran in die Darmzellen aufgenommen werden, um somit ihr komplexgebundenes Eisen direkt an das Ferritin oder an das Transferrin abzugeben oder direkt als intakter Komplex in die Blutbahn zu gelangen. Diese neuen Komplexe sollten aufgrund ihrer Eigenschaften praktisch nicht zum Auftreten von hohen Konzentrationen an freien Eisenionen führen. Denn gerade durch freie Eisenionen kommt es zum Auftreten von ROS, die letztlich für die auftretenden Nebenwirkungen verantwortlich sind.

Um diese Anforderungen erreichen zu können, entwickelten die Erfinder neue potente Fe(III)-Komplexverbindungen mit nicht zu grossem Molekulargewicht, mittlerer Lipophilie, sehr guter Aktivität bzw. Eisenutilisation, hoher Wasserlöslichkeit und optimaler pH-Wert abhängiger Komplexstabilität.

Bei der Entwicklung der neuen Komplexe sollte die Stabilitätsverbesserung gerade im neutralen wässrigen Medium nicht auf Kosten der Löslichkeit gehen, da die Löslichkeit für orale Anwendungen ein sehr wichtiges Kriterium darstellt. Dieses kombinierte Ziel wird mit den erfindungsgemäßen Fe-Komplexen erreicht. Diese zeigen eine gute Stabilität im wässrigen Medium bei neutralem pH-Wert und verfügen gleichzeitig über eine sehr gute Wasserlöslichkeit. Somit ermöglichen es die erfindungsgemäßen Eisen-Komplexverbindungen einen schnelleren Behandlungserfolg zu erreichen.

**Beschreibung der Erfindung:**

Die Erfinder fanden, dass gerade Fe(III)-Komplexverbindungen mit 3-Hydroxy-isonicotinamid -Liganden sich für die oben beschriebenen Anforderungen besonders eignen.

Die erfindungsgemäßen Eisen(III)-3-hydroxy-isonicotinamid-Komplexverbindungen können gleichbedeutend auch die Bezeichnung "Eisen(III)-3-hydroxy-N,N-dimethyl-pyridin-4-carboxamid-Komplex(verbindung)" oder "Eisen(III)-3-hydroxypyridin-4-carboxamid-Komplex(verbindung)" tragen und entsprechend können die erfindungsgemäßen 3-Hydroxy-isonicotinamid-Liganden gleichbedeutend auch als "3-Hydroxy-N,N-dimethyl-pyridin-4-carboxamid" oder "3-Hydroxypyridin-4-carboxamid" bzw. als entsprechender "-Ligand" bezeichnet werden.

Es konnte gezeigt werden, dass diese Fe-Komplexverbindungen eine hohe Eisenaufnahme zeigen, wodurch ein schneller Therapieerfolg bei der Behandlung der Eisenmangelanämie erreicht werden kann. Gerade im Vergleich mit Eisensalzen zeigen die erfindungsgemäßen Komplexverbindungen eine schnellere und höhere Utilisation. Ausserdem weisen diese neuen Systeme deutlich geringere Nebenwirkungen als die klassisch verwendeten Eisensalze auf, da es hier nicht in nennenswertem Ausmaß zum Auftreten von freien Eisenionen kommt. Die erfindungsgemäßen Komplexverbindungen zeigen nahezu keinen Oxidativen Stress, da es nicht zur Bildung von freien Radikalen kommt. Somit treten bei diesen Komplexverbindungen deutlich weniger Nebenwirkungen als bei den aus dem Stand der Technik

bekannten Fe Salzen auf. Die Komplexverbindungen zeigen sowohl im sauren als auch im neutralen pH-Wert-Bereich gute Stabilitäten was gerade für orale Anwendungen sehr vorteilhaft ist. Die Komplexverbindungen lassen sich gut herstellen und eignen sich optimal zur Formulierung von Arzneimitteln, insbesondere zur oralen Verabreichung.

Gegenstand der Erfindung sind somit Eisen(III)-3-hydroxy-isonicotinamid-Komplexverbindungen oder deren Salze, insbesondere pharmazeutisch verträgliche Salze zur Verwendung als Arzneimittel. Gegenstand der Erfindung sind demnach auch Eisen(III)-3-hydroxy-isonicotinamid-Komplexverbindungen oder deren pharmazeutisch verträglichen Salze zur Anwendung in einem Verfahren zur therapeutischen Behandlung des menschlichen oder tierischen Körpers.

Gegenstand der Erfindung sind außerdem die hierin definierten Eisen(III)-3-hydroxy-isonicotinamid-Komplexverbindungen *per se* (unabhängig von einer spezifischen medizinischen Verwendung).

Die erfindungsgemäß verwendeten Eisen(III)-3-hydroxy-isonicotinamid-Komplexverbindungen schließen insbesondere solche Verbindungen ein, die folgendes Strukturelement aufweisen:

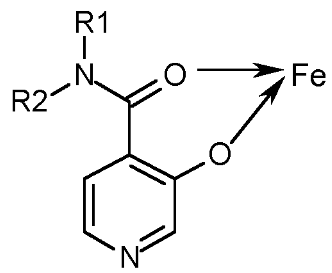

Die Begriffe

- "3-Hydroxy-isonicotinamid",
- "3-Hydroxy-isonicotinamid-Verbindungen" oder
- "3-Hydroxy-isonicotinamid-Liganden" sowie gleichbedeutend
- "3-Hydroxy-N,N-dimethyl-pyridin-4-carboxamid"
- "3-Hydroxy-N,N-dimethyl-pyridin-4-carboxamid-Verbindungen" oder
- "3-Hydroxy-N,N-dimethyl-pyridin-4-carboxamid-Liganden" bzw. gleichbedeutend
- "3-Hydroxypyridin-4-carboxamid",
- "3-Hydroxypyridin-4-carboxamid-Verbindungen", oder
- "3-Hydroxypyridin-4-carboxamid-Liganden"

schließen somit erfindungsgemäß sowohl die entsprechenden Hydroxy-Ausgangverbindungen

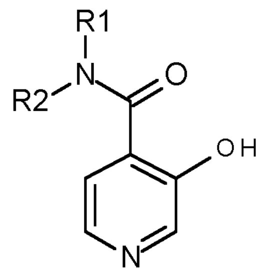

als auch die entsprechenden deprotonierten Liganden

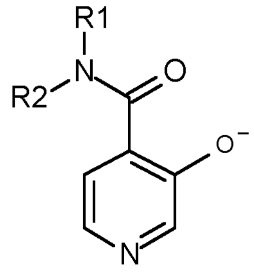

bzw.

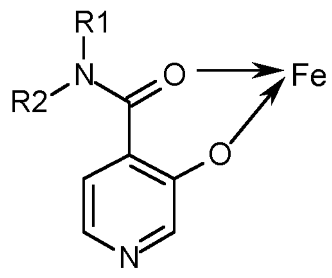

ein, welche in den entsprechenden Eisen(III)-Komplexverbindungen vorliegen.

Der Ligand geht dabei formal durch Abspalten eines Protons aus den entsprechenden 3-Hydroxy-isonicotinamid-Verbindungen hervor:

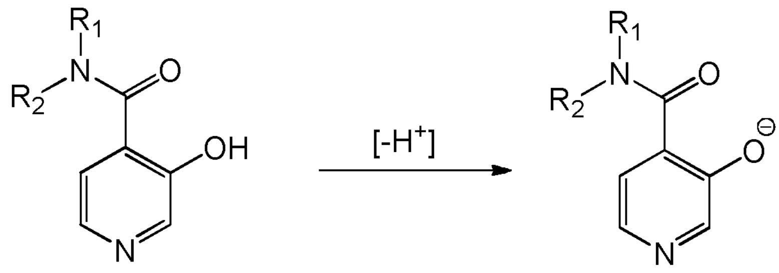


trägt also formal eine einfach negative Ladung.

Damit schließen die genannten Begriffe erfindungsgemäß den entsprechenden Grundkörper:

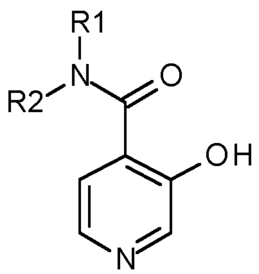

bzw. die durch Deprotonierung aus der zugrundeliegenden Hydroxyverbindung hervorgegangene Ligand-Verbindung

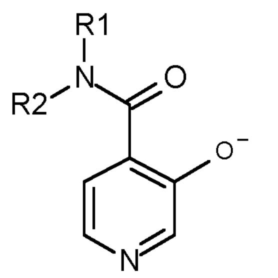

ein. Die genannten Begriffe bezeichnen somit erfindungsgemäß die Gesamtheit der Klasse der "3-Hydroxy-isonicotinamid"-Verbindungen bzw. der deprotonierten Liganden.

Ein (deprotonierter) 3-Hydroxy-isonicotinamid-Ligand wie er erfindungsgemäß verwendet wird, trägt somit, wie gezeigt, formal eine negative Ladung. Das bedeutet, dass bei drei Liganden je Eisenatom das Eisenatom formal die Oxidationsstufe +3 besitzt.

In den erfindungsgemäßen Eisen(III)-3-hydroxy-isonicotinamid-Komplexverbindungen beträgt die Koordinationszahl der Eisenatome damit im Allgemeinen sechs (6), wobei im Allgemeinen eine oktaedrische Anordnung der

koordinierenden Atome vorliegt. Damit liegen die erfindungsgemäßen Eisen(III)- 3-hydroxy-isonicotinamid-Komplexverbindungen bevorzugt als einkernige Komplexe vor, in denen ein (1) zentrales Eisenatom und drei (3) 3-Hydroxy-isonicotinamid-Liganden vorliegen.

Die erfindungsgemäßen Eisen(III)-3-hydroxy-isonicotinamid-Komplexverbindungen liegen im Allgemeinen in neutraler Form vor. Es sind jedoch auch salzartige Eisen(III)-3-hydroxy-isonicotinamid-Komplexverbindungen eingeschlossen, in denen der Komplex eine positive Ladung aufweist, die insbesondere durch pharmakologisch verträgliche, im wesentlichen nicht-koordinierende Anionen (wie insbesondere Halogenide, wie Chlorid) ausgeglichen wird.

Die erfindungsgemäßen Eisen(III)-3-hydroxy-isonicotinamid-Komplexverbindungen schließen insbesondere solche Komplexverbindungen ein, die mindestens einen, bevorzugt zweizähnigen 3-Hydroxy-isonicotinamid-Liganden der Formel

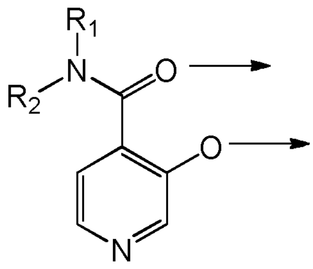

aufweisen, der, wie vorstehend gezeigt, an ein Eisenatom im Sinne einer Verbrückung gebunden sein kann.

Bevorzugt sind Eisen(III)-3-hydroxy-isonicotinamid-Komplexverbindungen, die ausschließlich, bevorzugt zweizähnige 3-Hydroxy-isonicotinamid-Liganden aufweisen, welche gleich oder verschieden sein können. Besonders bevorzugt sind weiterhin Eisen(III)-3-hydroxy-isonicotinamid-Komplexverbindungen, die ausschließlich die gleichen 3-Hydroxy-isonicotinamid-Liganden aufweisen, und ganz besonders bevorzugt sind Tris(3-hydroxy-isonicotinamid)-eisen(III)-Verbindungen.

In einer bevorzugten Ausführungsform enthalten die erfindungsgemäßen Eisen(III)-Komplexverbindungen drei gleiche oder verschiedene, bevorzugt gleiche, Liganden der Formel (I):

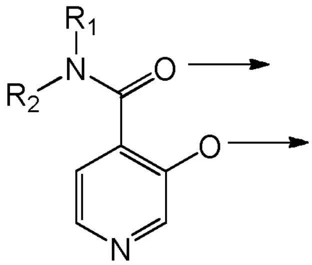

(I)

worin

die Pfeile jeweils eine koordinative Bindung zu einem oder verschiedenen, bevorzugt zu einem, Eisenatom darstellen; $R_1$ und $R_2$ gleich oder verschieden sind, und jeweils ausgewählt werden aus der Gruppe, die besteht aus:

- Wasserstoff und
- gegebenenfalls substituiertem Alkyl, oder worin

$R_1$ und $R_2$ gemeinsam mit dem Stickstoffatom, an welches sie gebunden sind, einen gegebenenfalls substituierten 3- bis 6-gliedrigen Ring bilden, der gegebenenfalls ein weiteres Heteroatom aufweisen kann,
oder pharmazeutisch verträgliche Salze davon.

Besonders bevorzugt sind insbesondere Eisen(III)-3-hydroxy-isonicotinamid-Komplexverbindungen, die mindestens einen Liganden der Formel (I) enthalten:

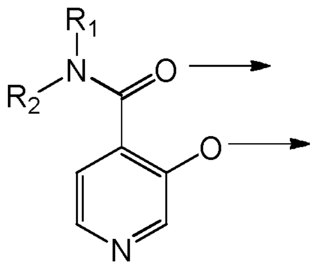

(I)

worin
die Pfeile jeweils eine koordinative Bindung zu einem oder verschiedenen, bevorzugt zu einem, Eisenatom darstellen; $R_1$ und $R_2$ gleich oder verschieden sind, und jeweils ausgewählt werden aus der Gruppe, die besteht aus:

- Wasserstoff und
- gegebenenfalls substituiertem Alkyl, oder worin

$R_1$ und $R_2$ gemeinsam mit dem Stickstoffatom, an welches sie gebunden sind, einen gegebenenfalls substituierten 5- bis 6-gliedrigen Ring bilden, der gegebenenfalls ein weiteres Heteroatom aufweisen kann,
pharmazeutisch verträgliche Salze davon.
Weitere besonders bevorzugte Ausführungsformen umfassen Eisen(III)-3-hydroxy-isonicotinamid-Komplexverbindungen, die mindestens einen Liganden der Formel (I) enthalten:

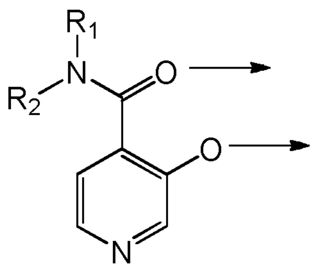

(I)

worin
die Pfeile jeweils eine koordinative Bindung zu einem oder verschiedenen, bevorzugt zu einem, Eisenatom darstellen; $R_1$ und $R_2$ gleich oder verschieden sind, und jeweils ausgewählt werden aus der Gruppe, die besteht aus:

- Wasserstoff und
- gegebenenfalls substituiertem Alkyl, umfassend insbesondere auch Alkyl, worin eine oder zwei Methylengruppen ($-CH_2-$) in den Alkylresten durch -O- ersetzt sein können, und welches gegebenenfalls mit einer oder mehreren Alkoxy- und/oder HydroxyGruppen substituiert ist; oder worin

$R_1$ und $R_2$ gemeinsam mit dem Stickstoffatom, an welches sie gebunden sind, einen gegebenenfalls substituierten 5- bis 6-gliedrigen Ring bilden, der gegebenenfalls ein weiteres Heteroatom aufweisen kann,
oder pharmazeutisch verträgliche Salze davon.
Noch bevorzugter sind Ausführungsformen umfassend Eisen(III)-3-hydroxy-isonicotinamid-Komplexverbindungen, die mindestens einen Liganden der Formel (I) enthalten:

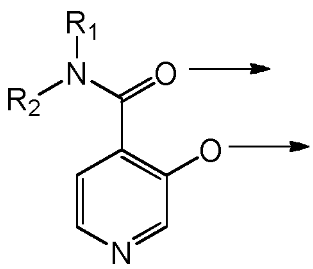

(I)

worin
die Pfeile jeweils eine koordinative Bindung zu einem Eisenatom darstellen;
$R_1$ und $R_2$ gleich oder verschieden sind, und jeweils ausgewählt werden aus der Gruppe, die besteht aus:

- Wasserstoff und
- Alkyl, umfassend insbesondere auch Alkyl, worin eine Methylengruppe ($-CH_2-$) durch -O- ersetzt sein kann, und welches gegebenenfalls mit einer oder mehreren AlkoxyGruppen substituiert ist, mit der Maßgabe, daß im Fall einer Alkyl-Gruppe, in der eine Methylengruppe ($-CH_2-$) durch -O- ersetzt ist, mindestens zwei Methylengruppen ($-CH_2-$) vor dieser -O- Gruppe und mindestens zwei Methylengruppen zwischen dieser und der -O- Gruppe des Alkoxy-Substituenten liegen, entsprechend einer Struktur gemäß $[(-CH_2-)_x-O-(CH_2-)_x-O-(CH_2)_yCH_3)]$, worin x jeweils eine ganze Zahl von 2 bis 4 und y ein ganze Zahl von 0 bis 2 darstellen;

oder pharmazeutisch verträgliche Salze davon.

Darunter sind ganz besonders bevorzugt Eisen(III)-3-hydroxy-isonicotinamid-Komplexverbindungen, die mindestens einen Liganden der Formel (I) enthalten:

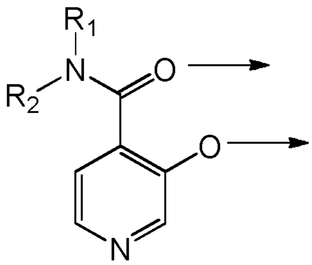

(I)

worin
die Pfeile jeweils eine koordinative Bindung zu einem oder verschiedenen, bevorzugt zu einem, Eisenatomen darstellen,
$R_1$ und $R_2$ gleich oder verschieden sind, und jeweils ausgewählt werden aus der Gruppe, die besteht aus:

- Wasserstoff,
- Methyl, Ethyl, Propyl, Isopropyl, n-Butyl, sek-Butyl Isobutyl,
- Methoxyethoxyethyl, Ethoxyethoxyethyl, Propoxyethoxyethyl,
- Methoxyethyl, Ethoxyethyl, Methoxypropyl und Ethoxypropyl; oder worin

$R_1$ und $R_2$ gemeinsam mit dem Stickstoffatom, an welches sie gebunden sind, einen gegebenenfalls substituierten heterocyclischen 5- bis 6-gliedrigen Ring bilden, der gegebenenfalls ein weiteres Heteroatom, bevorzugt ein Sauerstoffatom, aufweisen kann,
oder pharmazeutisch verträgliche Salze davon.

Besonders bevorzugte Eisen(III)-Komplexverbindungen enthalten mindestens einen Liganden der Formel (I):

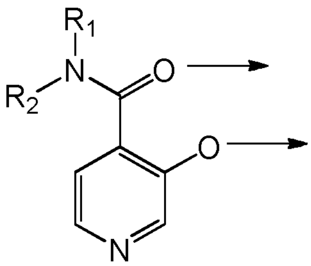

(I)

worin
die Pfeile jeweils eine koordinative Bindung zu einem oder verschiedenen Eisenatomen darstellen,
$R_1$ und $R_2$ gleich oder verschieden sind, und jeweils ausgewählt werden aus der Gruppe, die besteht aus Methyl, Ethyl, Propyl, n-Butyl und mit einer Methoxy-Gruppe oder einer Ethoxy-Gruppe, substituiertem Alkyl; oder worin
$R_1$ und $R_2$ gemeinsam mit dem Stickstoffatom, an welches sie gebunden sind, einen heterocyclischen 5- bis 6-gliedrigen Ring bilden, der ausgewählt ist aus der Gruppe bestehend aus Morpholinyl, Piperidinyl und Pyrrolidinyl,
oder pharmazeutisch verträgliche Salze davon.

Besonders bevorzugt sind Eisen(III)-Komplexverbindungen nach der Formel (II):

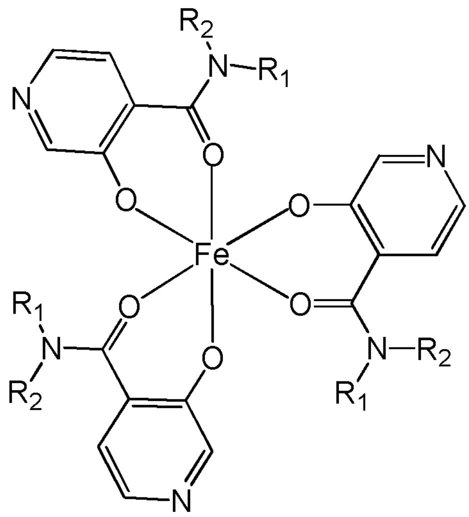

(II)

worin $R_1$ und $R_2$ wie vorstehend definiert sind.

Ganz besonders bevorzugt sind Eisen(III)-Komplexverbindungen, die ausgewählt sind aus der Gruppe:

| | |
|---|---|
| Tris-(3-Hydroxy-N,N-dimethylisonicotinamid)-eisen (III)-Komplex | 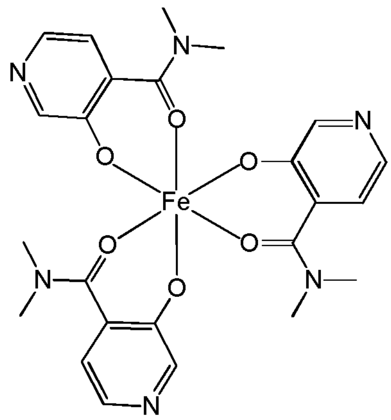 , |
| Tris-(3-Hydroxy-isonicotinamid)-eisen (III)-Komplex | 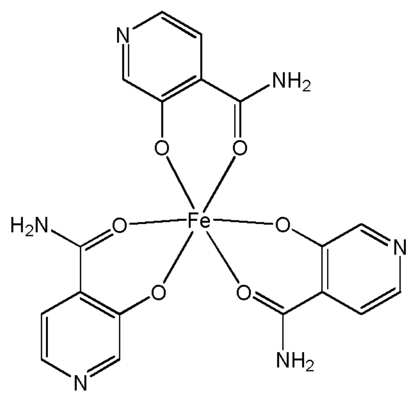 , |

(fortgesetzt)

| | |
|---|---|
| Tris-(3-Hydroxy-N-methylisonicotinamid)-eisen (III)-Komplex | 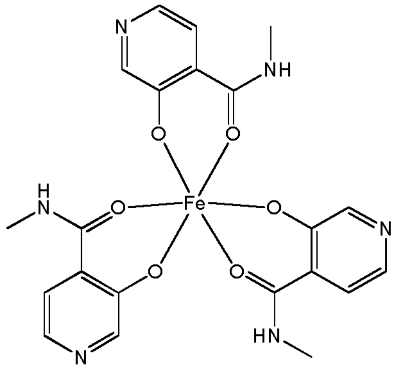 , |
| Tris-(3-Hydroxy-N-ethylisonicotinamid)-eisen(III)-Komplex | 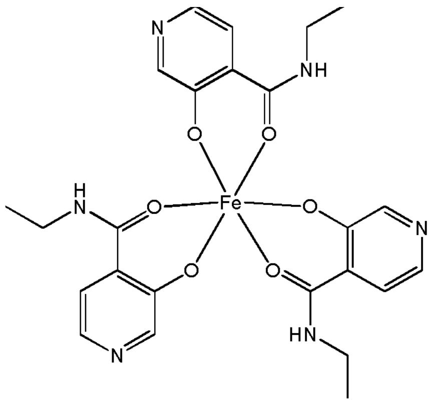 , |
| Tris-(3-Hydroxy-N-propylisonicotinamid)-eisen (III)-Komplex | 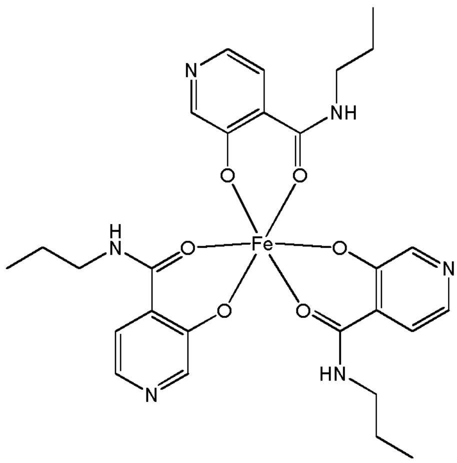 , |

(fortgesetzt)

| | |
|---|---|
| Tris-(N-Butyl-3-hydroxyisonicotinamid)-eisen (III)-Komplex | 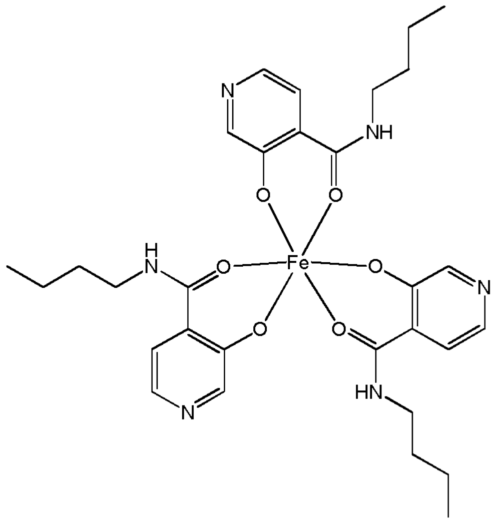 , |
| Tris-(3-Hydroxy-N-(2-methoxyethyl) isonicotinamid)-eisen(III)-Komplex | 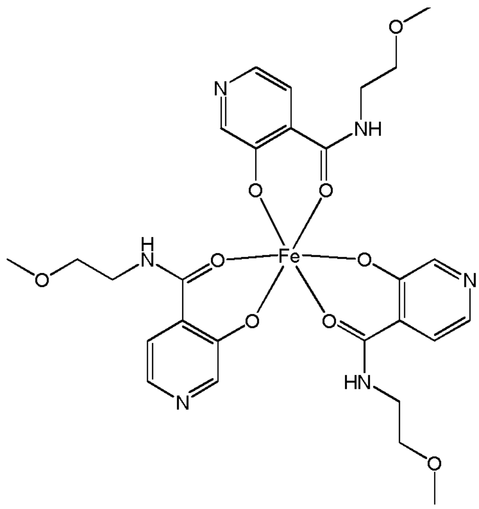 , |
| Tris-(3-Hydroxy-N-(2-methoxyethyl)-N-methylisonicotinamid)-eisen (III)-Komplex | 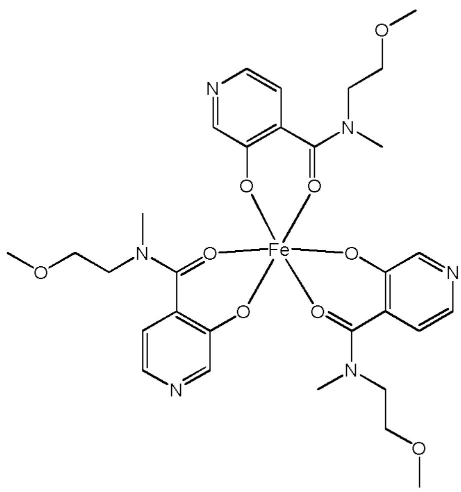 , |

(fortgesetzt)

| | |
|---|---|
| Tris-(3-Hydroxy-N, N-bis(2-methoxyethyl)isonicotinamid)-eisen (III)-Komplex | 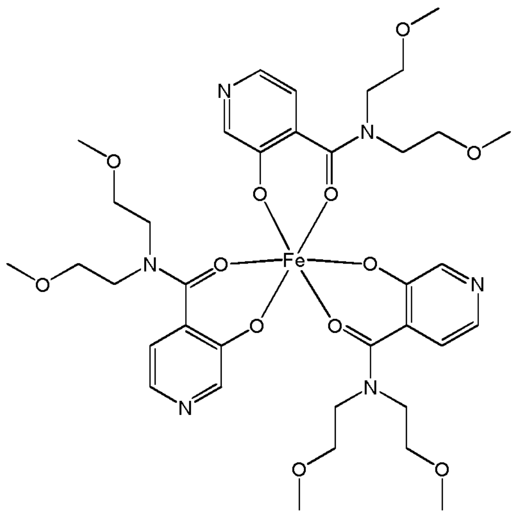 , |
| Tris-(N, N-Diethyl-3-hydroxyisonicotinamid)-eisen (III)-Komplex | 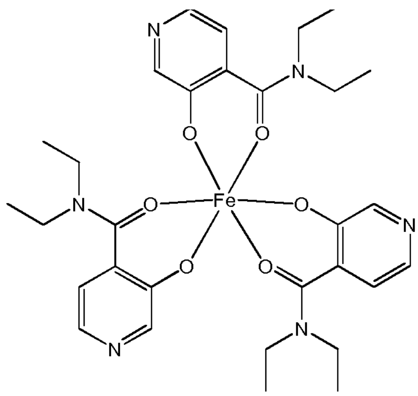 , |
| Tris-((3-Hydroxypyridin-4-yl) (morpholino)methanon)-eisen (III)-Komplex | 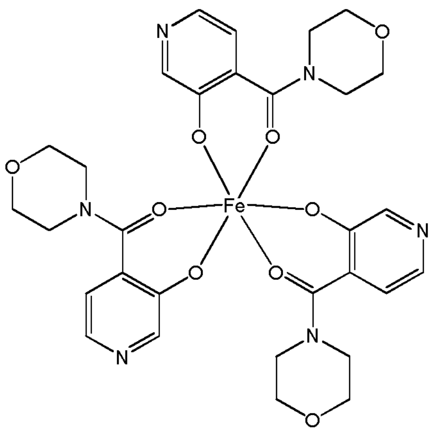 , |

(fortgesetzt)

| | |
|---|---|
| Tris-((3-Hydroxypyridin-4-yl)(piperidin-1-yl)methanon)-eisen(III)-Komplex | 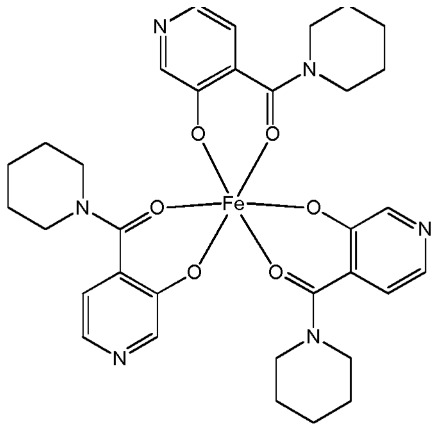 und |
| Tris-((3-Hydroxypyridin -4-yl)(pyrrolidin-1-yl)methanon)-eisen(III)-Komplex | 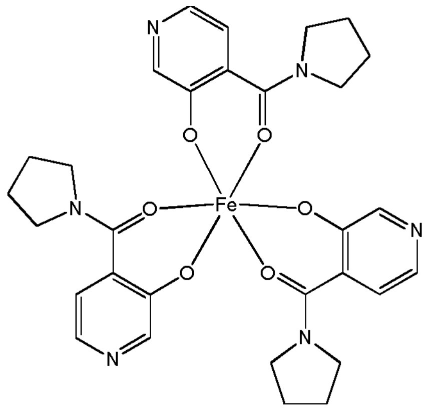 |

Bevorzugt liegt das Molekulargewicht der erfindungsgemäßen Eisen(III)-3-hydroxy-isonicotinamid Komplexverbindungen bei weniger als 1000 g/mol, noch bevorzugter bei weniger als 800 g/mol (jeweils bestimmt aus der Strukturformel).

Im Rahmen der gesamten Erfindung schließt gegebenenfalls substituiertes Alkyl insbesondere für die Substituenten $R_1$ und $R_2$ bevorzugt ein:
Geradkettiges oder verzweigtes Alkyl mit 1 bis 9, bevorzugt 1 bis 6, Kohlenstoffatomen, Cycloalkyl mit 3 bis 6, bevorzugt 5 oder 6 Kohlenstoffatomen, oder Alkyl mit 1 bis 4 Kohlenstoffatomen, welches mit Cycloalkyl substituiert ist, wobei diese Alkylgruppen jeweils gegebenenfalls substituiert sein können.

Die genannten Alkylgruppen können gegebenenfalls jeweils bevorzugt 1 bis 3 Substituenten tragen.

Die Substituenten von Alkyl werden bevorzugt aus der Gruppe ausgewählt, die besteht aus: Hydroxy und gegebenenfalls substituiertem Alkoxy insbesondere wie nachstehend definiert.

In den vorstehend definierten Alkylgruppen können des Weiteren gegebenenfalls ein oder zwei Kohlenstoffatome durch Sauerstoff ersetzt sein. Dies bedeutet insbesondere, dass eine oder zwei Methylengruppen ($-CH_2-$) in den Alkylresten durch -O- ersetzt sein können. Bevorzugt ist in solchen Ausgestaltungen eine Methylengruppe durch -O- ersetzt.

Beispiele von Alkylresten mit 1 bis 6 Kohlenstoffatomen schließen ein: eine Methylgruppe, eine Ethylgruppe, eine n-Propylgruppe, eine i-Propylgruppe, eine n-Butylgruppe, eine i-Butylgruppe, eine sec-Butylgruppe, eine t-Butylgruppe, eine n-Pentylgruppe, eine i-Pentylgruppe, eine sec-Pentylgruppe, eine t-Pentylgruppe, eine 2-Methylbutylgruppe, eine n-Hexylgruppe, eine 1-Methylpentylgruppe, eine 2-Methylpentylgruppe, eine 3-Methylpentylgruppe, eine 4-Methylpentylgruppe, eine 1-Ethylbutylgruppe, eine 2-Ethylbutylgruppe, eine 3-Ethylbutylgruppe, eine 1,1-Dimethylbutylgruppe, eine 2,2-Dimethylbutylgruppe, eine 3,3-Dimethylbutylgruppe, eine 1-Ethyl-1-methylpropylgruppe usw. Bevorzugt sind solche mit 1 bis 4 Kohlenstoffatomen. Am meisten bevorzugt sind Methyl, Ethyl, n-Propyl und n-Butyl.

Cycloalkylreste mit 3 bis 6 Kohlenstoffatomen schließen eine Cyclopropylgruppe, eine Cyclobutylgruppe, eine Cyclopentylgruppe und eine Cyclohexylgruppe ein. Die Cycloalkylreste können gegebenenfalls substituiert sein, bevorzugt mit einem Substituenten, wie Hydroxy, Akyl wie insbesondere Methyl und Ethyl, oder Alkoxy wie insbesondere Methoxy und Ethoxy.

Die Definition der gegebenenfalls substituierten Alkylgruppen schließt auch Alkylgruppen ein, welche durch

vorstehend definierte Cycloalkylgruppen substituiert sind, wie beispielsweise Cyclopropylmethyl, Cyclobutylmethyl, Cyclopentylmethyl oder Cyclohexylmethyl.

Beispiele eines mit Hydroxy substituierten Alkylrestes schließen die oben genannten Alkylreste ein, die 1 bis 2 Hydroxylreste aufweisen, wie zum Beispiel Hydroxymethyl, 2-Hydroxyethyl, 3-Hydroxypropyl, 4-Hydroxybutyl, etc.

Beispiele eines mit Alkoxy substituierten Alkylrestes schließen die oben genannten Alkylreste ein, die 1 bis 2 wie nachstehend definierte Alkoxyreste aufweisen, wie zum Beispiel Methoxymethyl, 2-Methoxyethyl, 3-Methoxypropyl, Ethoxymethyl, 2-Ethoxyethyl, 3-Ethoxypropyl etc.. Bevorzugt ist Methoxyethyl, Ethoxyethyl, Methoxypropyl und Ethoxypropyl, ganz besonders bevorzugt ist 2-Methoxyethyl.

Ebenfalls bevorzugt ist, daß ein Alkylrest, in dem eine Methylengruppe ($-CH_2-$) durch -O-ersetzt ist, mit einer Alkoxy-Gruppe, vorzugsweise Methoxy, Ethoxy oder Propoxy substituiert ist, mit der Maßgabe, daß mindestens zwei Methylengruppen ($-CH_2-$) vor und nach der resultierenden mittelständigen -O- Gruppe vorhanden sind, entsprechend $[(-CH_2-)_x-O-(CH_2-)_x-O-(CH_2)_yCH_3)]$, worin x eine ganze Zahl von 2 bis 4 und y ein ganze Zahl von 0 bis 2 darstellen. Entsprechende Beispiele umfassen insbesondere Methoxyethoxyethyl, Ethoxyethoxyethyl, Propoxyethoxyethyl, Methoxyethoxypropyl, Ethoxyethoxypropyl, Propoxyethoxypropyl, Methoxyethoxybutyl, Ethoxyethoxybutyl, Propoxyethoxybutyl, Methoxypropoxyethyl, Ethoxypropoxyethyl, Propoxypropoxyethyl, Methoxypropoxypropyl, Ethoxypropoxypropyl, Propoxypropoxypropyl, Methoxypropoxybutyl, Ethoxypropoxybutyl, Propoxypropoxybutyl, Methoxybutoxyethyl, Ethoxybutoxyethyl, Propoxybutoxyethyl, Methoxybutoxypropyl, Ethoxybutoxypropyl, Propoxybutoxypropyl, Methoxybutoxybutyl, Ethoxybutoxybutyl, Propoxybutoxybutyl; bevorzugt sind Methoxyethoxyethyl, Ethoxyethoxyethyl und Propoxyethoxyethyl

Gegebenenfalls substituiertes Alkoxy (RO-) leitet sich formal durch Hinzufügen eines Sauerstoffatom zu einem der vorstehend erwähnten gegebenenfalls substituierten Alkylreste ab, und schließt erfindungsgemäß beispielsweise lineare oder verzweigte Alkoxyreste bevorzugt mit bis zu 4 Kohlenstoffatomen ein, wie eine Methoxygruppe, eine Ethoxygruppe, eine n-Propyloxygruppe und eine i-Propyloxygruppe etc. ein.

Die Alkoxygruppen können gegebenenfalls substituiert sein, wie beispielsweise mit den für Alkyl genannten möglichen Substituenten, insbesondere mit 1 bis 3, bevorzugter 1 Substituenten.

Bevorzugtes Alkoxy ist Methoxy, Ethoxy und n-Propoxy, besonders bevorzugt ist Methoxy und Ethoxy, ganz besonders bevorzugt ist Methoxy.

Im Rahmen der vorliegenden Erfindung können die Substituenten $R_1$ und $R_2$ gemeinsam mit dem Stickstoffatom, an welches sie gebunden sind, einen gegebenenfalls substituierten 3- bis 6-gliedrigen Ring, bevorzugt einen gegebenenfalls substituierten 5-bis 6-gliedrigen Ring bilden, der gegebenenfalls ein weiteres Heteroatom, vorzugsweise ein Sauerstoffatom, aufweisen kann. Bevorzugt bilden darin $R_1$ und $R_2$ gemeinsam mit dem Stickstoffatom einen aliphatischen Ring. Beispiele schließen erfindungsgemäß ein gesättigte 3- bis 6-gliedrige, bevorzugt 5- oder 6-gliedrige, heterocyclische Ringe, welche über das Stickstoffatom angebunden sind, und welche gegebenenfalls ein weiteres Heteroatom wie insbesondere O oder N, bevorzugt O, aufweisen können, wie zum Beispiel Aziridinyl (Aziridin-1-yl), substituiertes Aziridin-1-yl, Azetidinyl (Azetidin-1-yl), substituiertes Azetidin-1-yl, Pyrrolidinyl (Pyrrolidin-1-yl), substituiertes Pyrrolidin-1-yl, Piperidinyl, wie Piperidin-1-yl, substituiertes Piperidin-1-yl, Imidazolinyl (Imidazolin-1-yl), substituiertes Imidazolin-1-yl, Piperazinyl (Piperazin-1-yl), substituiertes Piperazin-1-yl, Morpholinyl, substituiertes Morpholinyl. Bevorzugt bilden $R_1$ und $R_2$ gemeinsam mit dem Stickstoffatom einen Pyrrolidinyl, Piperidinyl oder Morpholinylrest.

Die Erfindung betrifft weiterhin ein Verfahren zur Herstellung der erfindungsgemäßen Eisen(III)-Komplexverbindungen, welches die Umsetzung eines 3-Hydroxyisonicotinamids (III), entsprechend der protonierten Form des erfindungsgemäßen Liganden, mit einem Eisen(III)-salz (IV) umfasst.

3-Hydroxy-isonicotinamide schließen insbesondere diejenigen der Formel (III) ein:

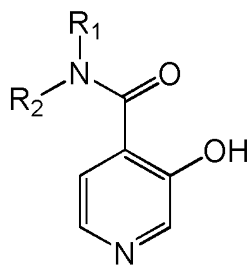

(III)

worin $R_1$ und $R_2$ wie oben definiert sind.

Beispiele geeigneter Eisen(III)-salze schließen ein: Eisen(III)-chlorid, Eisen(III)-acetat, Eisen(III)-sulfat, Eisen(III)-nitrat und Eisen(III)-acetylacetonat, worunter Eisen(III)-chlorid bevorzugt ist.

Ein bevorzugtes Verfahren ist im folgenden Schema gezeigt:

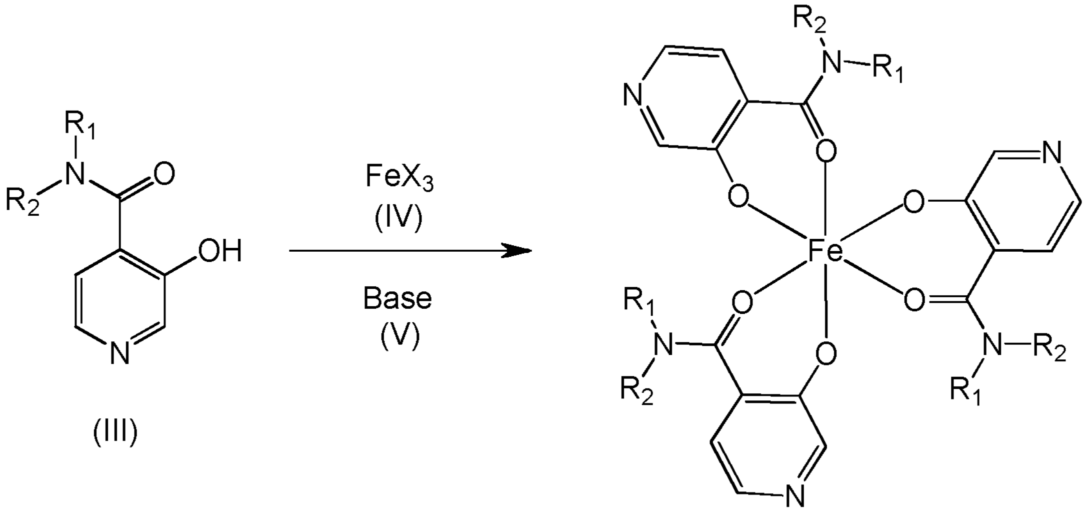


(III)

(II)

worin $R_1$ und $R_2$ wie oben definiert sind, X ein Anion wie Halogenid, wie Chlorid, ein Carboxylat, wie Acetat, Sulfat, Nitrat und Acetylacetonat ist und Base eine übliche organische oder anorganische Base darstellt.

Beim erfindungsgemäßen Verfahren werden bevorzugt 3-5 eq Ligand (III) unter Verwendung von geeigneten Eisen(III)-salzen (IV) (besonders eignen sich hier Fe(III)-chlorid, Fe(III)-acetat, Fe(III)-sulfat und Fe(III)-acetylacetonat) zu den entsprechenden Komplexen, der allgemeinen Formel (II), unter Standardbedingungen umgesetzt. Hierbei wird die Synthese unter den für die Komplexbildung optimalen pH-Bedingungen durchgeführt. Der optimale pH-Wert wird gegebenenfalls durch Zugabe von Base (V), besonders eignet sich hier die Verwendung von Triethylamin, Natrium-Carbonat, Natrium-Hydrogencarbonat, Natrium-Methanolat, Natrium-Ethanolat, Kaliumcarbonat, Kalium-Hydrogencarbonat oder Kalium-Methanolat, eingestellt.

Die für die Darstellung der Komplexe benötigten Liganden (III) sind entweder käuflich erhältlich oder wurden nach folgender Synthesemethode dargestellt. Hierzu wurde nachfolgender Syntheseweg beschritten.

Der als Ausgangsverbindung benötigte 3-Hydroxyisonicotinsäureethylester (VI) wurde analog Crum und Fuchsman (J. Heterocyclic Chem. 1966, 3, 252-256) hergestellt und mit verschiedenen Aminen der allgemeinen Formel (VII) gemäss der allgemeinen Reaktionsgleichung zu den entsprechenden 3-Hydroxy-isonicotinamiden der allgemeinen Formel (III) umgesetzt. Alternativ können auch 3-Hydroxyisonicotinsäurehalogenide oder aktivierte 3-Hydroxyisonicotinsäureester unter für den Fachmann vertrauten Reaktionsbedingungen zu den gewünschten Verbindungen der allgemeinen Formel (III) umgesetzt werden.

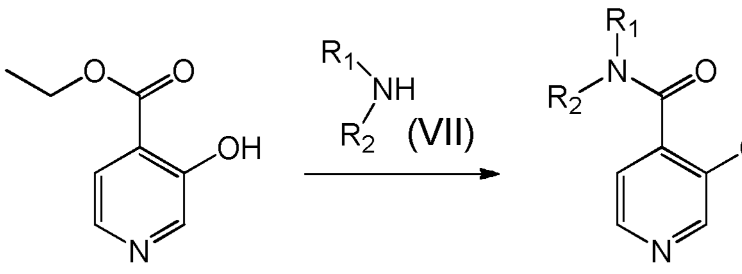


(VI) (III)

Pharmazeutisch annehmbare Salze der erfindungsgemäßen Verbindungen, in denen der Eisen(III)-Komplex formal eine positive Ladung trägt, schließen beispielsweise Salze mit geeigneten Anionen ein, wie Carboxylate, Sulfonate, Sulfate, Chloride, Bromide, Iodide, Phosphate, Tartrate, Methansulfonate, Hydroxyethansulfonate, Glycinate, Maleate, Propionate, Fumarate, Toluolsulfonate, Benzolsulfonate, Trifluoracetate, Naphthalindisulfonate-1,5, Salicylate, Benzoate, Lactate, Salze der Äpfelsäure, Salze der 3-Hydroxy-2-naphthoesäure-2, Citrate und Acetate ein.

Pharmazeutisch annehmbare Salze der erfindungsgemäßen Verbindungen, in denen der Eisen(III)-Komplex formal eine negative Ladung trägt, schließen beispielsweise Salze mit geeigneten pharmazeutisch annehmbaren Basen ein, wie z.B. Salze mit Alkali- oder Erdalkalihydroxiden, wie NaOH, KOH, $Ca(OH)_2$, $Mg(OH)_2$ etc., Aminverbindungen,

wie Ethylamin, Diethylamin, Triethylamin, Ethyldiisopropylamin, Ethanolamin, Diethanolamin, Triethanolamin, Methylglucamin, Dicyclohexylamin, Dimethylaminoethanol, Prokain, Dibenzylamin, N-Methylmorpholin, Arginin, Lysin, Ethylendiamin, N-Methylpiperidin 2-Amino-2-methyl-propanol-(1), 2-Amino-2-methyl-propandiol-(1,3) , 2-Amino-2-hydroxylmethyl-propandiol-(1,3) (TRIS) etc..

Die Wasserlöslichkeit bzw. die Löslichkeit in physiologischer Kochsalzlösung und damit gegebenenfalls auch die Wirksamkeit der erfindungsgemäßen Verbindungen kann durch Salzbildung generell, speziell auch durch die Wahl des Gegenions signifikant beeinflusst werden.

Bevorzugt stellen die erfindungsgemäßen Verbindungen neutrale Komplex-Verbindungen dar.

**Vorteilhafte pharmakologische Effekte:**

Die Erfinder fanden überraschend, dass die Eisen(III)-3-hydroxyisonicotinamid-Komplexverbindungen, die Gegenstand der vorliegenden Erfindung sind, und die insbesondere durch die allgemeine Strukturformel (II) dargestellt werden, stabile bioverfügbare Eisenkomplexe sind und zur Verwendung als Arzneimittel zur Behandlung und Prophylaxe von Eisenmangelerscheinungen und Eisenmangelanämien sowie der damit einhergehenden Symptome geeignet sind.

Die erfindungsgemäßen Verbindungen enthaltende Arzneimittel eignen sich zum Einsatz in der Human- und der Veterinärmedizin.

Die erfindungsgemäßen Verbindungen eignen sich somit auch zur Herstellung eines Medikamentes zur Behandlung von Patienten, die unter Symptomen einer Eisenmangelanämie wie zum Beispiel: Ermüdung, Antriebslosigkeit, Konzentrationsschwäche, geringe kognitive Effizienz, Schwierigkeiten beim Finden der richtige Worte, Vergesslichkeit, unnatürliche Blässe, Reizbarkeit, Beschleunigung der Herzfrequenz (Tachykardie), wunde oder geschwollene Zunge, vergrößerte Milz, Schwangerengelüste (Pica), Kopfschmerzen, Appetitlosigkeit, erhöhte Infektionsanfälligkeit oder einer depressive Verstimmungen leiden.

Die erfindungsgemäßen Eisen(III)-Komplexverbindungen eignen sich weiterhin zur Behandlung der Eisenmangelanämie bei Schwangeren, der latenten Eisenmangelanämie bei Kindern und Heranwachsenden, des Eisenmangels und der Eisenmangelanämie bei Kindern mit Aufmerksamkeitsdefizit-/Hyperaktivitätsstörung (ADHS), der Eisenmangelanämie infolge gastrointestinaler Abnormalitäten, der Eisenmangelanämie infolge von Blutverlusten, wie durch gastrointestinale Blutungen (z.B. infolge von Geschwüren, Karzinomen, Hämorriden, entzündlichen Störungen, Einnahme von Acetylsalicylsäure), der Eisenmangelanämie infolge von Menstruation, der Eisenmangelanämie infolge von Verletzungen, der Eisenmangelanämie infolge von Psilosis (sprue), der Eisenmangelanämie infolge verminderter Eisenaufnahme bei der Ernährung, insbesondere bei selektiv essenden Kindern und Heranwachsenden, der Immunschwäche hervorgerufen durch Eisenmangelanämie, der Beeinträchtigung der Hirnleistung hervorgerufen durch Eisenmangelanämien, des Restless Leg Syndrom hervorgerufen durch Eisenmangelanämien, Eisenmangelanämien bei Krebs, Eisenmangelanämien ausgelöst durch Chemotherapien, Eisenmangelanämien ausgelöst durch Inflammation (AI), Eisenmangelanämien bei kongestiver Herzinsuffizienz (CHF;congestive heart failure), Eisenmangelanämien bei chronischer Niereninsuffizienz Stadium 3 -5 (CKD 3-5; chronic kidney diseases stage 3-5), Eisenmangelanämien ausgelöst durch chronische Inflammation (ACD), Eisenmangelanämien bei rheumatischer Arthritis (RA; rheumatoid arthritis), Eisenmangelanämien bei systemischem Lupus erythemotodes (SLE; systemic lupus erythematosus) und Eisenmangelanämien bei inflammatorischen Darmerkrankungen (IBD; inflammatory bowel diseases).

Die Verabreichung kann über einen Zeitraum von mehreren Monaten bis zur Verbesserung des Eisenstatus, reflektiert beispielsweise durch den Hämoglobin-Wert, die Transferrin-Sättigung und den Serum Ferritin-Wert der Patienten, oder zur gewünschten Verbesserung einer durch Eisenmangelanämie hervorgerufenen Beeinträchtigung des Gesundheitszustandes erfolgen.

Das erfindungsgemäße Präparat kann von Kindern, Jugendlichen und Erwachsenen eingenommen werden.

Die erfindungsgemäßen Verbindungen angewendeten Verbindungen können dabei sowohl oral als auch parenteral verabreicht werden. Bevorzugt ist die orale Verabreichung.

Die erfindungsgemäßen Verbindungen sowie die vorgenannten Kombinationen der erfindungsgemäßen Verbindungen mit weiteren Wirkstoffen oder Arzneimitteln können somit insbesondere zur Herstellung von Medikamenten zur Behandlung der Eisenmangelanämie verwendet werden, wie Eisenmangelanämien bei Schwangeren, der latenten Eisenmangelanämie bei Kindern und Heranwachsenden, der Eisenmangelanämie infolge gastrointestinaler Abnormalitäten, der Eisenmangelanämie infolge von Blutverlusten, wie durch gastrointestinale Blutungen (z.B. infolge von Geschwüren, Karzinomen, Hämorriden, entzündlichen Störungen, Einnahme von Acetylsalicylsäure), Menstruation, Verletzungen, Eisenmangelanämie infolge von Psilosis (sprue), Eisenmangelanämie infolge verminderter Eisenaufnahme bei der Ernährung, insbesondere bei selektiv essenden Kindern und Heranwachsenden, Immunschwäche hervorgerufen durch Eisenmangelanämie, Beeinträchtigung der Hirnleistung hervorgerufen durch Eisenmangelanämie, Restless Leg Syndrom.

Die erfindungsgemäße Anwendung führt zu einer Verbesserung der Eisen-, Hämoglobin-, Ferritin- und Transferrinwerte, welche insbesondere bei Heranwachsenden und Kindern aber auch bei Erwachsenen mit einer Verbesserung im Kurzzeitgedächtnistests (STM), im Langzeitgedächtnistest (LTM), im Test der progressiven Matrices nach Raven, in der Welschers Erwachsenenintelligenzskala (WAIS) und/oder im emotionalen Koeffizienten (Baron EQ-i, YV-Test; Jugendversion), oder zu einer Verbesserung der Neutrophilen-Niveaus, der Antikörperniveaus und/oder der Lymphozytenfunktion einhergeht.

Die vorliegende Erfindung betrifft weiterhin pharmazeutische Zusammensetzungen, enthaltend eine oder mehrere der erfindungsgemäßen Verbindungen, insbesondere nach Formel (II), sowie gegebenenfalls eine oder mehrere weitere pharmazeutisch wirksame Verbindung sowie gegebenenfalls einen oder mehrere pharmakologisch verträgliche Träger und/oder Hilfsstoffe und/oder Lösungsmittel. Die genannten pharmazeutische Zusammensetzungen enthalten beispielsweise bis 99 Gew.-% oder bis zu 90 Gew.-% oder bis zu 80 Gew.-% oder bis zu 70 Gew.-% der erfindungsgemäßen Verbindungen, wobei der Rest jeweils durch pharmakologisch verträgliche Träger und/oder Hilfsstoffe und/oder Lösungsmittel und/oder gegebenenfalls weitere pharmazeutisch wirksame Verbindungen gebildet wird.

Dabei handelt es sich um übliche pharmazeutische Träger, Hilfsstoff oder Lösungsmittel. Genannte pharmazeutische Zusammensetzungen sind beispielsweise geeignet zur intravenösen, intraperitonealen, intramuskulären, intravaginalen, intrabuccalen, perkutanen, subkutanen, mucokutanen, oralen, rektalen, transdermalen, topikalen, intradermalen, intragastralen oder intrakutanen Applikation und liegen beispielsweise in der Form von Pillen, Tabletten, magensaftresistenten Tabletten, Filmtabletten, Schichttabletten, Retardformulierungen zur oralen, subkutanen oder kutanen Verabreichung (insbesondere als Pflaster), Depotformulierung, Dragees, Zäpfchen, Gelen, Salben, Sirup, Granulaten, Suppositorien, Emulsionen, Dispersionen, Mikrokapseln, Mikroformulierungen, Nanoformulierungen, liposomalen Formulierungen, Kapseln, magensaftresistente Kapseln, Pulvern, Inhalationspulvern, mikrokristallinen Formulierungen, Inhalationssprays, Puder, Tropfen, Nasentropfen, Nasensprays, Aerosolen, Ampullen, Lösungen, Säften, Suspensionen, Infusionslösungen oder Injektionslösungen etc. vor.

In einer bevorzugten Ausführungsform der Erfindung werden die Eisen-Komplexverbindungen in Form einer Tablette oder Kapsel verabreicht. Diese können beispielsweise als säureresistente Formen oder mit pH-abhängigen Beschichtungen vorliegen.

Gegenstand der vorliegenden Erfindung sind somit auch Zusammensetzungen, die die erfindungsgemäßen Eisen(III)-Komplexverbindungen enthalten, in Kombination mit mindestens einer weiteren pharmazeutisch wirksamen Verbindung. Beispiele derartiger weiterer pharmazeutisch wirksamer Verbindungen umfassen insbesondere bekannte Wirkstoffe oder Arzneimittel, die begleitend mit Mitteln zur Behandlung von mit Eisenmetabolismus-Störungen assoziierten Erkrankungen, insbesondere solchen, die mit Eisenmangel und/oder Anämien (Eisenmangelanämien) assoziiert sind, verabreicht werden, sowie solche Verbindungen, die auf den Eisenmetabolismus wirken und somit bevorzugt in der Behandlung von Eisenmetabolismus-Störungen eingesetzt werden. Beispiele solcher in Kombination anwendbarer Mittel zur Behandlung von Eisenmetabolismus-Störungen und weiterer mit Eisenmangel und/oder Anämien assoziierter Erkrankungen können beispielsweise umfassen Vitamin C und Vitamin D und/oder Derivate davon.

Bevorzugt werden die erfindungsgemäßen Verbindungen sowie pharmazeutische Zusammensetzungen, enthaltend solche Verbindungen oral appliziert obwohl auch andere Formen wie parenteral, insbesondere intravenös möglich sind.

Dazu liegen die erfindungsgemäßen Verbindungen bevorzugt in pharmazeutischen Zusammensetzungen in Form von Pillen, Tabletten, magensaftresistenten Tabletten, Filmtabletten, Schichttabletten, Retardformulierungen zur oralen Verabreichung, Depotformulierungen, Dragees, Granulaten, Emulsionen, Dispersionen, Mikrokapseln, Mikroformulierungen, Nanoformulierungen, liposomalen Formulierungen, Kapseln, magensaftresistente Kapseln, Pulvern, mikrokristallinen Formulierungen, Puder, Tropfen, Ampullen, Lösungen, Suspensionen, Infusionslösungen oder Injektionslösungen vor.

Die erfindungsgemäßen Verbindungen können in pharmazeutischen Zusammensetzung verabreicht werden, die verschiedene organische oder anorganische Träger- und/oder Hilfsmaterialien enthalten können, wie sie üblicherweise für pharmazeutische Zwecke insbesondere für feste Arzneimittelformulierungen verwendet werden, wie beispielsweise Exzipienten (wie Saccharose, Stärke, Mannit, Sorbit, Lactose, Glucose, Cellulose, Talk, Calciumphosphat, Calciumcarbonat), Bindemittel (wie Cellulose, Methylcellulose, Hydroxypropylcellulose, Polypropylpyrrolidon, Gelatine, Gummiarabicum, Polyethylenglykol, Saccharose, Stärke), Desintegrationsmittel (wie Stärke, hydrolysierte Stärke, Carboxymethylcellulose, Calciumsalz von Carboxymethylcellulose, Hydroxypropylstärke, Natriumglycolstärke, Natriumbicarbonat, Calciumphosphat, Calciumcitrat), Gleit- bzw. Schmiermittel (wie Magnesiumstearat, Talk, Natriumlaurylsulfat), ein Geschmacksbildner (wie Citronensäure, Menthol, Glycin, Orangenpulver), Konservierungsmittel (wie Natriumbenzoat, Natriumbisulfit, Methylparaben, Propylparaben), Stabilisatoren (wie Citronensäure, Natriumcitrat, Essigsäure, und Multicarbonsäuren aus der Titriplex Reihe wie z.B. Diethylentriaminpentaessigsäure (DTPA), Suspendiermittel (wie Methylcellulose, Polyvinylpyrrolidon, Aluminiumstearat), Dispergiermittel, Verdünnungsmittel (wie Wasser, organische Lösungsmittel), Bienenwachs, Kakaobutter, Polyethylenglykol, weißes Petrolatum etc..

Flüssige Arzneimittelformulierungen, wie Lösungen, Suspensionen und Gele enthalten üblicherweise einen flüssigen Träger, wie Wasser und/oder pharmazeutisch verträgliche organische Lösungsmittel. Weiterhin können derartige flüssigen Formulierungen auch pH-einstellende Mittel, Emulgatoren oder dispergierende Agenzien, puffernde

Agenzien, Konservierungsmittel, Netzmittel, Geliermittel (beispielsweise Methylcellulose), Färbemittel und/oder Aromastoffe enthalten. Die Zusammensetzungen können isotonisch sein, das heisst diese können den gleichen osmotischen Druck wie Blut haben. Die Isotonie der Zusammensetzung kann durch die Verwendung von Natriumchlorid oder anderer pharmazeutisch annehmbare Agenzien wie beispielsweise Dextrose, Maltose, Borsäure, Natriumtartrat, Propylenglykol oder andere anorganische oder organisch lösliche Substanzen eingestellt werden. Die Viskosität der flüssigen Zusammensetzungen kann unter Verwendung eines pharmazeutisch annehmbaren Verdickungsmittels, wie Methylcellulose eingestellt werden. Andere geeignete Verdickungsmittel umfassen beispielsweise Xanthan, Carboxymethylcellulose, Hydroxypropylcellulose, Carbomer und dergleichen. Die bevorzugte Konzentration des Verdickungsmittels wird von dem ausgewählten Agens abhängen. Pharmazeutisch annehmbare Konservierungsmittel können verwendet werden, um die Haltbarkeit der flüssigen Zusammensetzung zu erhöhen. Benzylalkohol kann geeignet sein, obwohl eine Vielzahl von Konservierungsmitteln einschließlich beispielsweise Paraben, Thimerosal, Chlorbutanol oder Benzalkoniumchlorid ebenfalls verwendet werden können.

Der Wirkstoff kann beispielsweise mit einer Einheitsdosis von 0,001 mg/kg bis 500 mg/kg Körpergewicht beispielweise bis zu 1 bis 4 mal am Tag verabreicht werden. Die Dosierung kann jedoch je nach Alter, Gewicht, Zustand des Patienten, Schwere der Erkrankung oder Art der Verabreichung erhöht oder herabgesetzt werden.

## BEISPIELE

Die Erfindung wird durch die folgenden Beispiele näher veranschaulicht. Die Beispiele stellen lediglich Exemplifizierungen dar, und der Fachmann ist in der Lage, die spezifischen Beispiele auf weitere beanspruchte Verbindungen auszudehnen. Die Bezeichnung der Beispielnamen wurde mit Hilfe des Computerprogrammes ChemDraw Ultra Version 12.0 bestimmt und festgelegt.

## AUSGANGSVERBINDUNGEN:

Die in den Beispielen verwendeten Ausgangsverbindungen wurden wie folgt erhalten.

### A. 3-Hydroxy-*N,N*-dimethylisonicotinamid

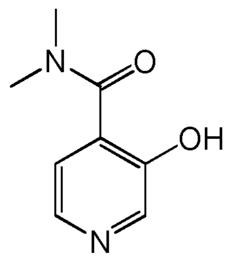

0.09 mol (15 g) 3-Hydroxyisonicotinsäureethylester (analog J.D. Crum, C.H. Fuchsman, J. Heterocyclic Chem. 1966, 3, 252-256) und 0.9 mol Dimethylamin (160 ml 5.6 M Lösung in Ethanol) wurden im Druckbehälter 5 h auf 120°C geheizt. Anschliessend wurde das Reaktionsgemisch zur Trockne eingeengt und das Rohprodukt aus 200 ml Tetrahydrofuran und 100 ml Ethanol umkristallisiert. Man erhielt 10 g (67% Ausbeute) der Titelverbindung.
IR (in Substanz, $cm^{-1}$): 1626, 1601, 1573, 1501, 1444, 1418, 1394, 1300, 1263, 1238, 1202, 1166, 1084, 1066, 964, 934, 838, 780, 741, 711, 645.
LC-MS (m/z): 167.5 (M+H).
CHN-Elementaranalyse: C, 58.22; H, 6.20; N, 16.72.
1H-NMR (DMSO-$d_6$, 400 MHz): $\delta$ [ppm] = 10.29 (s, 1H), 8.22 (s, 1H), 8.06 (d, 1H), 7.11 (d, 1H), 2.95 (s, 3H), 2.77 (s, 3H).

### B. 3-Hydroxyisonicotinamid

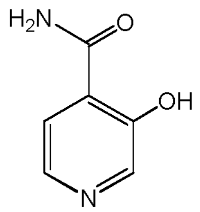

0.072 mol (12 g) 3-Hydroxyisonicotinsäureethylester und 0.72 mol Ammoniak (103 ml 7 M Lösung in Methanol) wurden im Druckbehälter 5 h auf 120°C geheizt. Anschliessend wurde das Reaktionsgemisch zur Trockne eingeengt, mit 165 ml Tetrahydrofuran aufgeschlämmt und 30 min auf Rückfluss erhitzt. Nach dem Abkühlen wurde das Produkt abfiltriert und getrocknet. Man erhielt 5 g (50% Ausbeute) der Titelverbindung.
IR (in Substanz, $cm^{-1}$): 1699, 1665, 1638, 1597, 1561, 1498, 1440, 1418, 1349, 1310, 1250, 1227, 1197, 1096, 1063, 1027, 911, 788, 767, 672, 653, 623.
LC-MS (m/z): 139.4 (M+H).
CHN-Elementaranalyse: C, 50.92; H, 4.21; N, 19.16.
1H-NMR (DMSO-$d_6$, 400 MHz): $\delta$ [ppm] = 8.52 (s, 1H), 8.37 (s, 1H), 8.21 (s, 1H), 8.18 (d, 1H), 7.80 (d, 1H).

**C. 3-Hydroxy-*N*-methylisonicotinamid**

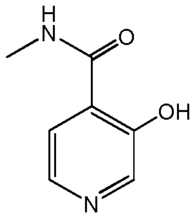

0.072 mol (12 g) 3-Hydroxyisonicotinsäureethylester und 0.25 mol Dimethylamin (31 ml 33% Lösung in Ethanol) wurden im Druckbehälter 3 h auf 120°C geheizt. Anschliessend wurde das Reaktionsgemisch zur Trockne eingeengt. Das Rohprodukt wurde aus 130 ml tert-Butylmethylester und 120 ml Ethanol umkristallisiert und anschliessend chromatografisch über Kieselgel mit Essigsäureethylester/Methanol 9/1 plus 1% Essigsäure als Eluenten gereinigt. Man erhielt 6 g (55% Ausbeute) der Titelverbindung.
IR (in Substanz, $cm^{-1}$): 1646, 1539, 1428, 1405, 1360, 1184, 1060, 1019, 893, 859, 818, 785, 664.
LC-MS (m/z): 153.8 (M+H).
CHN-Elementaranalyse: C, 55.18; H, 5.46; N, 18.36.
1H-NMR (DMSO-$d_6$, 400 MHz): $\delta$ [ppm] = 12.17 (s, 1H), 8.94 (breit, 1H), 8.34 (s, 1H), 8.15 (d, 1H), 7.69 (d, 1H), 2.85 (d, 3H).

**D. 3-Hydroxy-*N*-ethylisonicotinamid**

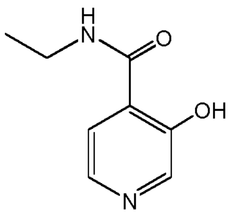

0.072 mol (12 g) 3-Hydroxyisonicotinsäureethylester und 0.22 mol Ethylamin (108 ml 2 M Lösung in Methanol) wurden im Druckbehälter 2 h auf 100°C geheizt. Anschliessend wurde das Reaktionsgemisch zur Trockne eingeengt und das Rohprodukt aus 400 ml Essigsäureethylester und 20 ml Ethanol umkristallisiert. Man erhielt 7.8 g (65% Ausbeute) der Titelverbindung.
IR (in Substanz, $cm^{-1}$): 1643, 1527, 1481, 1443, 1353, 1293, 1211, 1150, 1060, 843, 814, 783, 663.
CHN-Elementaranalyse: C, 57.68; H, 6.15; N, 16.81.

1H-NMR (DMSO-$d_6$, 400 MHz): δ [ppm] = 12.21 (s, 1H), 8.97 (m, 1H), 8.34 (s, 1H), 8.15 (d, 1H), 7.72 (d, 1H), 3.35 (m, 2H), 1.16 (t, 3H).

**E. 3-Hydroxy-*N*-propylisonicotinamid**

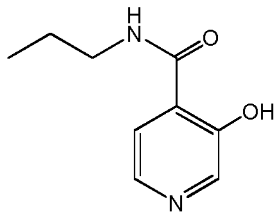

0.072 mol (12 g) 3-Hydroxyisonicotinsäureethylester und 0.3 mol n-Propylamin (24.5 ml) wurden im Druckbehälter 2 h auf 100°C geheizt. Anschliessend wurde das Reaktionsgemisch zur Trockne eingeengt und das Rohprodukt chromatografisch über Kieselgel mit Essigsäureethylester/Methanol 4/1 als Eluenten gereinigt. Man erhielt 6.4 g (49% Ausbeute) der Titelverbindung.
IR (in Substanz, $cm^{-1}$): 1640, 1597, 1542, 1492, 1462, 1416, 1322, 1304, 1217, 1150, 1128, 1061, 979, 914, 815, 786, 735, 667.
LC-MS (m/z): 181.5 (M+H).
CHN-Elementaranalyse: C, 56.33; H, 6.57; N, 14.27.
1H-NMR (DMSO-$d_6$, 400 MHz): δ [ppm] = 12.17 (breit, 1H), 8.96 (m, 1H), 8.33 (s, 1H), 8.14 (d, 1H), 7.72 (d, 1H), 3.28 (q, 2H), 1.56 (hextett, 2H), 0.90 (t, 3H).

**F. *N*-Butyl-3-hydroxyisonicotinamid**

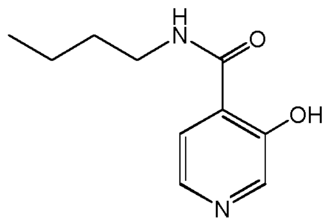

0.054 mol (9 g) 3-Hydroxyisonicotinsäureethylester und 0.27 mol *n*-Butylamin (26.7 ml) wurden 3 h auf 90°C geheizt. Anschliessend wurde überschüssiges *n*-Butylamin unter Vakuum abdestilliert und das Rohprodukt chromatografisch über Kieselgel mit Essigsäureethylester/Methanol 4/1 als Eluenten gereinigt. Man erhielt 7.7 g (74% Ausbeute) der Titelverbindung.
IR (in Substanz, $cm^{-1}$): 1632, 1599, 1549, 1465, 1416, 1334, 1211, 1177, 1117, 1056, 951, 921, 878, 829, 788, 734, 701, 660, 599, 557.
LC-MS (m/z): 195.1 (M+H).
CHN-Elementaranalyse: C, 60.33; H, 7.76; N, 14.32.
1H-NMR (DMSO-de, 400 MHz): δ [ppm] = 12.2 (breit, 1H), 8.92 (s, 1H), 8.30 (s, 1H), 8.12 (d, 1H), 7.70 (d, 1H), 3.29 (q, 2H), 1.52 (quintett, 2H), 1.31 (sextett, 2H), 0.88 (t, 3H).

**G. 3-Hydroxy-*N*-(2-methoxyethyl)isonicotinamid**

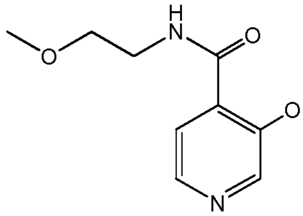

0.072 mol (12 g) 3-Hydroxyisonicotinsäureethylester und 0.36 mol (31.2 ml) 2-Methoxyethylamin wurden 3 h zum Rückfluss erhitzt. Anschliessend wurde überschüssiges 2-Methoxyethylamin unter Vakuum abdestilliert und das Rohprodukt chromatografisch über Kieselgel mit dem Eluenten Essigsäureethylester/Methanol 9/1 gereinigt. Man erhielt 9.3 g (66% Ausbeute) der Titelverbindung.
IR (in Substanz, $cm^{-1}$): 1645, 1592, 1537, 1493, 1454, 1428, 1409, 1346, 1326, 1308, 1243, 1224, 1194, 1163, 1131, 1115, 1092, 1065, 1051, 1017, 908, 887, 818, 795, 671.
LC-MS (m/z): 197 (M+H).
CHN-Elementaranalyse: C, 54.91; H, 6.14; N, 14.26.
1H-NMR (DMSO-$d_6$, 400 MHz): $\delta$ [ppm] = 12.08 (s, 1H), 8.99 (s, 1H), 8.34 (s, 1H), 8.15 (d, 1H), 7.75 (d, 1H), 3.50 (m, 4H), 3.29 (s, 3H).

**H. 3-Hydroxy-*N*-(2-methoxyethyl)-*N*-methylisonicotinamid**

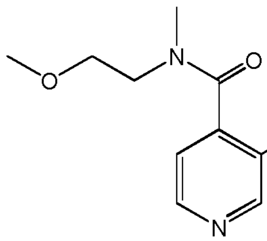

0.072 mol (12 g) 3-Hydroxyisonicotinsäureethylester und 0.223 mol (20.8 ml) N-(2-Methoxyethyl)methylamin wurden 3 h zum Rückfluss erhitzt. Anschliessend wurde überschüssiges 2-Methoxyethylamin unter Vakuum abdestilliert und das Rohprodukt chromatografisch über Kieselgel mit dem Eluenten Essigsäureethylester/Methanol 9/1 gereinigt. Man erhielt 14 g (92% Ausbeute) der Titelverbindung.
IR (in Substanz, $cm^{-1}$): 1633, 1600, 1569, 1478, 1453, 1415, 1397, 1309, 1296, 1264, 1247, 1237, 1195, 1164, 1127, 1088, 1063, 976, 932, 895, 837, 793, 778, 742, 721, 643, 613.
LC-MS (m/z): 211.6 (M+H).
CHN-Elementaranalyse: C, 56.93; H, 6.70; N, 13.28.
1H-NMR (DMSO-$d_6$, 400 MHz): $\delta$ [ppm] = 10.27 (s, 1H), 1.23/1.22 (s, 1H), 8.08/8.07 (d, 1H), 7.12/7.11 (d, 1H), 3.59/3.54 (t, 2H), 3.36/3.25 (t, 2H), 3.29/3.14 (s, 3H), 2.98/2.82 (s, 3H), (doppelter Signalsatz im Verhältnis 53:47 durch E/Z-Isomerie).

**1. 3-Hydroxy-*N,N*-bis(2-methoxyethyl)isonicotinamid**

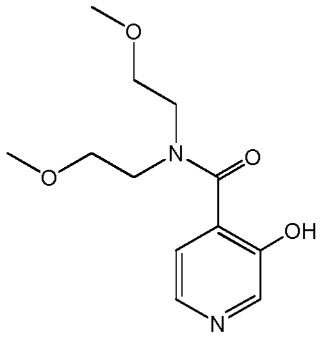

0.084 mol (14 g) 3-Hydroxyisonicotinsäureethylester und 0.108 mol (14.38 g) Bis-(2-methoxyethyl)amin wurden 6 h auf 110°C erhitzt. Anschliessend wurde das Rohprodukt chromatografisch über Kieselgel mit dem Eluenten Essigsäureethylester/Methanol 5/1 gereinigt. Man erhielt 6.19 g (30% Ausbeute) der Titelverbindung.
IR (in Substanz, $cm^{-1}$): 1633, 1597, 1463, 1416, 1364, 1306, 1232, 1202, 1181, 1114, 1066, 1015, 828, 777, 744, .
LC-MS (m/z): 255.5 (M+H).
CHN-Elementaranalyse: C, 54.14; H, 7.30; N, 10.49.
1H-NMR (DMSO-$d_6$, 400 MHz): $\delta$ [ppm] = 10.25 (s, 1H), 8.21 (s, 1H), 8.07 (d, 1H), 7.10 (d, 1H), 3.61 (t, 2H), 3.52 (t, 2H), 3.34 (t, 2H), 3.29 (m, 5H), 3.12 (s, 3H).

**J. *N,N*-Diethyl-3-hydroxyisonicotinamid**

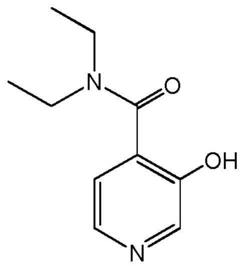

0.072 mol (12.4 g) 3-Hydroxyisonicotinsäureethylester und 0.49 mol Diethylamin (51 ml) wurden im Druckbehälter 3 h bei 110°C gerührt. Anschliessend wurde das Reaktionsgemisch zur Trockne eingeengt. Das Rohprodukt wurde in 20 ml Essigsäureethylester gelöst, nach Zugabe von 20 ml Diethylether 1 h gerührt und abfiltriert, der Filterrückstand wurde getrocknet. Man erhielt 11.3g (80% Ausbeute) der Titelverbindung.
IR (in Substanz, $cm^{-1}$): 1630, 1564, 1500, 1479, 1458, 1431, 1383, 1360, 1313, 1298, 1288, 1253, 1222, 1197, 1167, 1095, 1069, 945, 907, 881, 862, 832, 783, 737, 699, 636, 605.
LC-MS (m/z): 195.2 (M+H).
CHN-Elementaranalyse: C, 61.58; H, 7.15; N, 14.43.
1H-NMR (DMSO-$d_6$, 400 MHz): $\delta$ [ppm] = 10.20 (s, 1H), 8.22 (s, 1H), 8.07 (d, 1H), 7.11 (d, 1H), 3.43 (q, 2H), 3.08 (q, 2H), 1.13 (t, 3H), 0.99 (t, 3H).

**K. (3-Hydroxypyridin-4-yl)(morpholino)methanon**

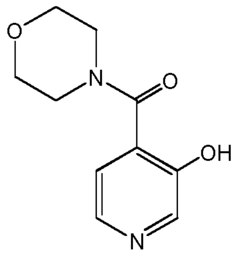

0.07 mol (12 g) 3-Hydroxyisonicotinsäureethylester und 0.288 mol Morpholin (25 ml) wurden 4 h bei 100°C gerührt. Anschliessend wurde überschüssiges Morpholin unter mehrfacher Zugabe von Toluol abdestilliert und das Rohprodukt aus 140 ml Essigsäureethylester und 130 ml Ethanol umkristallisiert. Man erhielt 11.5 g (79% Ausbeute) der Titelverbindung.
IR (in Substanz, $cm^{-1}$): 1642, 1606, 1576, 1507, 1451, 1429, 1361, 1328, 1304, 1276, 1266, 1234, 1177, 1140, 1111, 1062, 1016, 907, 896, 858, 830, 773, 742, 712, 648, 623.
LC-MS (m/z): 209.7 (M+H).
CHN-Elementaranalyse: C, 57.69; H,5.80 ; N, 13.47.
1H-NMR (DMSO-$d_6$, 400 MHz): $\delta$ [ppm] = 10.35 (s, 1H), 8.23 (s, 1H), 8.09 (d, 1H), 7.17 (d, 1H), 3.62 (m, 4H), 3.53 (t, 2H), 3.16 (t, 2H).

**L. (3-Hydroxypyridin-4-yl)(piperidin-1-yl)methanon**

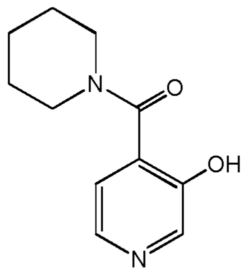

0.072 mol (12 g) 3-Hydroxyisonicotinsäureethylester und 0.39 mol Piperidin (40 ml) wurden 3 h bei 100°C gerührt. Anschliessend wurde überschüssiges Piperidin unter Zugabe von Ethanol abdestilliert. Der Rückstand wurde in 100 ml Essigsäureethylester suspendiert, 1 h gerührt und ausgefallenes Produkt abfiltriert. Nach dem Trocknen erhielt man 13 g (88% Ausbeute) der Titelverbindung.
IR (in Substanz, $cm^{-1}$): 1631, 1606, 1573, 1505, 1427, 1363, 1351, 1303, 1280, 1252, 1232, 1184, 1126, 1109, 1063, 1029, 1002, 951, 931, 909, 885, 847, 822, 772, 742, 707, 639, 617.
LC-MS (m/z): 207.6 (M+H).
CHN-Elementaranalyse: C, 64.08; H, 6.862; N, 13.59.
1H-NMR (DMSO-$d_6$, 400 MHz): $\delta$ [ppm] = 10.23 (s, 1H), 8.21 (s, 1H), 8.06 (d, 1H), 7.12 (d, 1H), 3.57 (m, 2H), 3.11 (m, 2H), 1.59 (m, 2H), 1.52 (m, 2H), 1.44 (m, 2H).

**M. (3-Hydroxypyridin-4-yl)(pyrrolidin-1-yl)methanon**

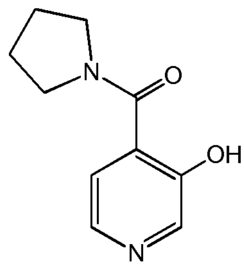

0.072 mol (12 g) 3-Hydroxyisonicotinsäureethylester und 0.48 mol Pyrrolidin (40 ml) wurden 3 h unter Rückfluss erhitzt. Anschliessend wurde überschüssiges Pyrrolidin mehrfach unter Zugabe von Ethanol abdestilliert. Der Rückstand wurde in 100 ml Essigsäureethylester suspendiert, 1 h gerührt und das Produkt abfiltriert. Nach dem Trocknen erhielt man 8.1 g (42% Ausbeute) der Titelverbindung.
IR (in Substanz, $cm^{-1}$): 1575, 1470, 1452, 1414, 1383, 1336, 1306, 1258, 1223, 1200, 1179, 1067, 856, 831, 796, 775, 735, 654.
LC-MS (m/z): 193.6 (M+H).
CHN-Elementaranalyse: C, 62.27; H, 6.57; N, 14.47.
1H-NMR (DMSO-$d_6$, 400 MHz): $\delta$ [ppm] = 10.33 (s, 1H), 8.25 (s, 1H), 8.08 (d, 1H), 7.16 (d, 1H), 3.44 (d, 2H), 3.20 (d, 2H), 1.81 (m, 4H).

**HERSTELLUNGSBEISPIELE:**

**Beispiel 1**

**Tris-(3-Hydroxy-*N,N*-dimethylisonicotinamid)-eisen(III)-Komplex:**

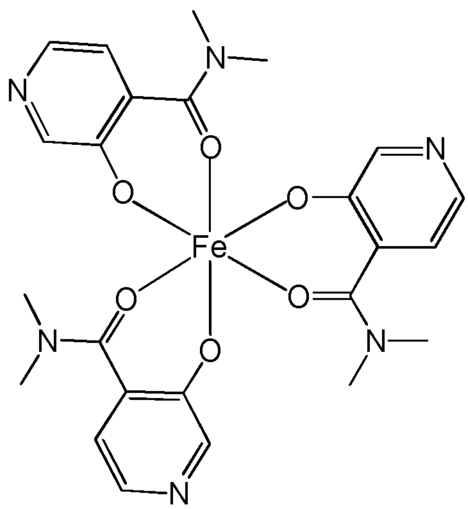

72 mmol (12 g) 3-Hydroxy-*N,N*-dimethylisonicotinamid wurden in 250 ml Ethanol vorgelegt und 72 mmol Natriummethanolat (30% Lösung in Methanol) zugegeben. Es wurden 24 mmol (3.89 g) $FeCl_3$ (wasserfrei) in 20 ml Ethanol gelöst zugetropft und 2 h gerührt. Das Reaktionsgemisch wurde filtriert, das Filtrat am Rotationsverdampfer eingeengt und der Rückstand getrocknet. Man erhielt 13.6 g (96% Fe-Ausbeute) der Titelverbindung.
IR (in Substanz, $cm^{-1}$): 1574, 1525, 1473, 1394, 1308, 1273, 1236, 1206, 1161, 1059, 928, 867, 850, 826, 790, 732, 711, 652, 615.
CHN-Elementaranalyse: C, 51.11; H, 5.44; N, 14.37.
Fe-Gehalt: 9.47% [m/m]

**2. Tris-(3-Hydroxyisonicotinamid)-eisen(III)-Komplex**

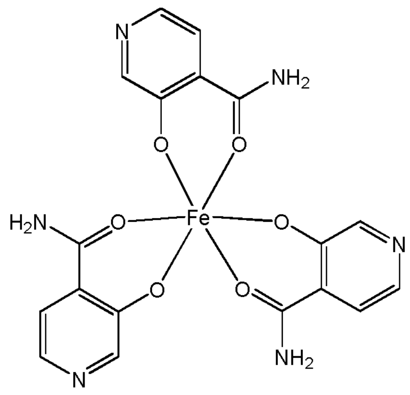

9 mmol (1.37 g) 3-Hydroxyisonicotinamid wurden in 60 ml Methanol unter leichtem Erwärmen gelöst und 3 mmol (0.49 g) $FeCl_3$ (wasserfrei) sowie 9 mmol Natriummethanolat (1.67 ml einer 30% Lösung in Methanol) zugegeben. Es wurde 0.5 h gerührt, das Reaktionsgemisch auf die Hälfte eingeengt und filtriert. Das Filtrat wurde am Rotationsverdampfer zur Trockne eingeengt und der Rückstand bei 50°C im Vakuumtrockenschrank getrocknet. Man erhielt 1.84 g (96% Fe-Ausbeute) der Titelverbindung.
IR (in Substanz, $cm^{-1}$): 1644, 1596, 1561, 1477, 1445, 1395, 1320, 1221, 1102, 1065, 1012, 904, 877, 827, 787, 761, 651, 618.
CHN-Elementaranalyse: C, 35.75; H, 2.92; N, 13.53.
Fe-Gehalt: 8.71% [m/m]
Chlorid-Gehalt: 12.1% [m/m]

**3. Tris-(3-Hydroxy-*N*-methylisonicotinamid)-eisen(III)-Komplex**

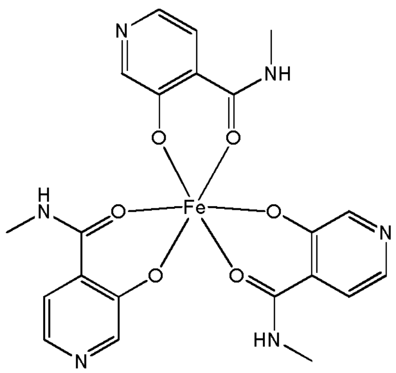

9 mmol (1.37 g) 3-Hydroxy-*N*-methylisonicotinamid wurden in 20 ml Ethanol gelöst und 3 mmol Natriummethanolat (2.06 ml 25% Lösung in Methanol) zugegeben. Es wurden 3 mmol (0.487 g) $FeCl_3$ (wasserfrei) in 10 ml Ethanol gelöst zugetropft und 2 h gerührt. Das Reaktionsgemisch wurde filtriert, das Filtrat am Rotationsverdampfer eingeengt und der Rückstand bei 50°C im Vakuumtrockenschrank getrocknet. Man erhielt 1.61 g (95% Fe-Ausbeute) der Titelverbindung.
IR (in Substanz, $cm^{-1}$): 1590, 1548, 1474, 1406, 1319, 1217, 1155, 1125, 1067, 966, 904, 881, 832, 784, 665.
CHN-Elementaranalyse: C, 47.21; H, 4.73; N, 15.38.
Fe-Gehalt: 9.91% [m/m]
Chlorid-Gehalt: 0.64% [m/m]

**4. Tris-(3-Hydroxy-*N*-ethylisonicotinamid)-eisen(III)-Komplex**

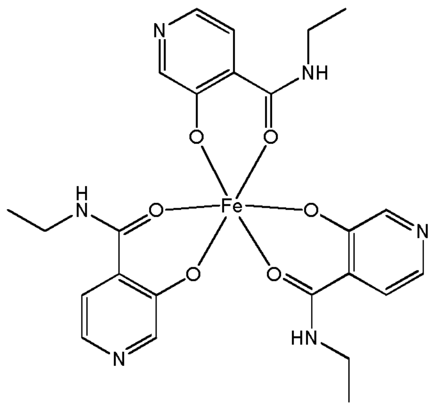

9 mmol (1.5 g) 3-Hydroxy-*N*-ethylisonicotinamid wurden in 20 ml Ethanol vorgelegt und 9 mmol Natriummethanolat (2.06 ml 25% Lösung in Methanol) zugegeben. Es wurden 3 mmol (0.49 g) $FeCl_3$ (wasserfrei) in 10 ml Ethanol gelöst zugetropft und 2 h gerührt. Das Reaktionsgemisch wurde filtriert, das Filtrat am Rotationsverdampfer eingeengt und der Rückstand bei 50°C im Vakuumtrockenschrank getrocknet. Man erhielt 1.71 g (96% Fe-Ausbeute) der Titelverbindung.
IR (in Substanz, $cm^{-1}$): 1583, 1546, 1473, 1403, 1321, 1264, 1216, 1146, 1128, 1070, 1037, 898, 838, 784, 698, 665.
CHN-Elementaranalyse: C, 48.07; H, 4.99; N, 13.97.
Fe-Gehalt: 9.43% [m/m]
Chlorid-Gehalt: 2.92% [m/m]

**5. Tris-(3-Hydroxy-*N*-propylisonicotinamid)-eisen(III)-Komplex**

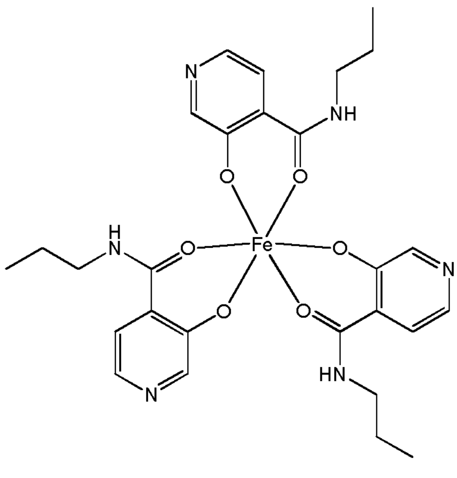

15 mmol (2.78 g) 3-Hydroxy-*N*-propylisonicotinamid wurden in 70 ml Ethanol vorgelegt und 15 mmol Natriummethanolat (3.43 ml 25% Lösung in Methanol) zugegeben. Es wurden 5 mmol (0.84 g) $FeCl_3$ (wasserfrei) in 10 ml Ethanol gelöst zugetropft und 2 h gerührt. Das Reaktionsgemisch wurde filtriert, das Filtrat am Rotationsverdampfer eingeengt und der Rückstand bei 50°C im Vakuumtrockenschrank getrocknet. Man erhielt 2.95 g (99% Fe-Ausbeute) der Titelverbindung.
IR (in Substanz, $cm^{-1}$): 1582, 1549, 1473, 1437, 1403, 1323, 1270, 1246, 1216, 1145, 1125, 1069, 871, 825, 786, 699, 666.
CHN-Elementaranalyse: C, 50.87; H, 5.68; N, 13.10.
Fe-Gehalt: 9.41% [m/m]
Chlorid-Gehalt: 2.5% [m/m]

**6. Tris-(*N*-Butyl-3-hydroxyisonicotinamid)-eisen(III)-Komplex**

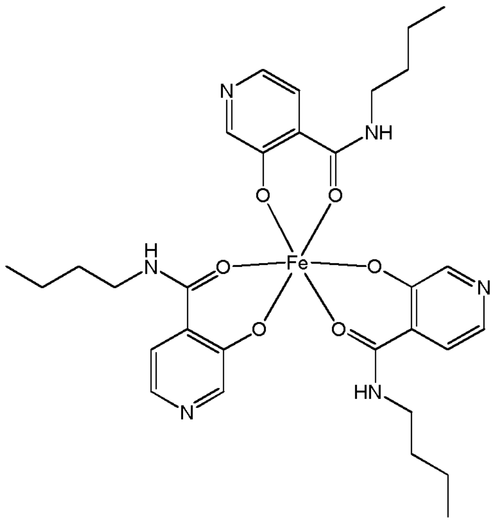

15 mmol (2.9 g) *N*-Butyl-3-hydroxyisonicotinamid wurden in 50 ml Ethanol vorgelegt und 5 mmol (0.81 g) in 5 ml Ethanol gelöstes $FeCl_3$ (wasserfrei) zugegeben sowie 30 mmol Natriumhydrogencarbonat. Es wurde 3 h gerührt, dann das Reaktionsgemisch filtriert, das Filtrat am Rotationsverdampfer eingeengt und der Rückstand bei 50°C im Vakuumtrockenschrank getrocknet. Man erhielt 3.1 g (92% Fe-Ausbeute) der Titelverbindung.
IR (in Substanz, $cm^{-1}$): 1581, 1548, 1472, 1436, 1403, 1323, 1215, 1125, 1069, 979, 950, 836, 785, 740, 698, 666, 622, 590, 553, 526, 507.
CHN-Elementaranalyse: C, 54.80; H, 6.03; N, 12.70.
Fe-Gehalt: 8.32% [m/m]
Chlorid-Gehalt: 1.2% [m/m]

**7. Tris-(3-Hydroxy-*N*-(2-methoxyethyl)isonicotinamid)-eisen(III)-Komplex**

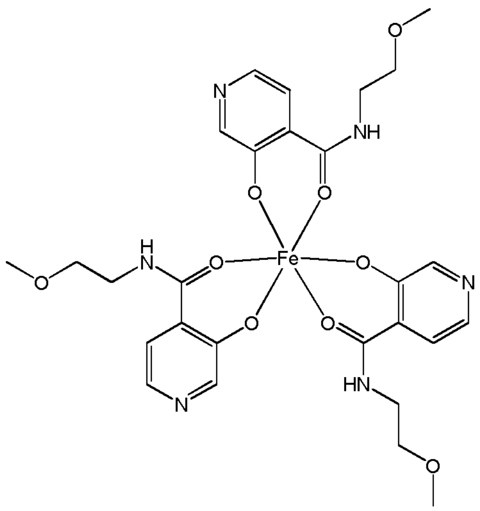

15.8 mmol (3.1 g) 3-Hydroxy-*N*-(2-methoxyethyl)isonicotinamid wurden in 50 ml Ethanol vorgelegt und 15 mmol (0.81 g) Natriummethanolat gelöst in 5 ml Methanol zugegeben. Es wurden 5.24 mmol (0.85 g) $FeCl_3$ (wasserfrei) gelöst in 10 ml Ethanol zugetropft und 2 h gerührt. Das Reaktionsgemisch wurde filtriert, das Filtrat am Rotationsverdampfer eingeengt und der Rückstand bei 50°C im Vakuumtrockenschrank getrocknet. Man erhielt 3.1 g (85% Fe-Ausbeute) der

Titelverbindung.
IR (in Substanz, $cm^{-1}$): 1585, 1550, 1475, 1404, 1363, 1323, 1218, 1196, 1117, 1018, 962, 893, 826, 784, 671.
CHN-Elementaranalyse: C, 46.77; H, 5.38; N, 11.98.
Fe-Gehalt: 8.14% [m/m]
Chlorid-Gehalt: 3.2% [m/m]

**8. Tris-(3-Hydroxy-*N*-(2-methoxyethyl)-N-methylisonicotinamid)-eisen(III)-Komplex**

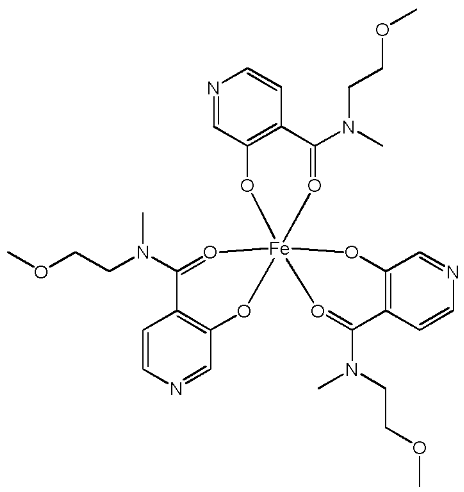

15 mmol (3.25 g) 3-Hydroxy-*N*-(2-methoxyethyl)-*N*-methylisonicotinamid wurden in 50 ml Ethanol vorgelegt und 15 mmol Natriummethanolat (25% Lösung in Methanol) zugegeben. Es wurden 5 mmol (0.84 g) $FeCl_3$ (wasserfrei) in 10 ml Ethanol gelöst zugetropft und 2 h gerührt. Das Reaktionsgemisch wurde filtriert, das Filtrat am Rotationsverdampfer eingeengt und der Rückstand bei 50°C im Vakuumtrockenschrank getrocknet. Man erhielt 3.4 g (98% Fe-Ausbeute) der Titelverbindung.
IR (in Substanz, $cm^{-1}$): 1563, 1523, 1472, 1396, 1309, 1230, 1204, 1157, 1113, 1063, 1015, 976, 910, 823, 790, 729, 707, 650, 614.
CHN-Elementaranalyse: C, 50.32; H, 5.97; N, 11.60.
Fe-Gehalt: 8.18% [m/m]
Chlorid-Gehalt: 1.3% [m/m]

**9. Tris-(3-Hydroxy-*N*,*N*-bis(2-methoxyethyl)isonicotinamid)-eisen(III)-Komplex**

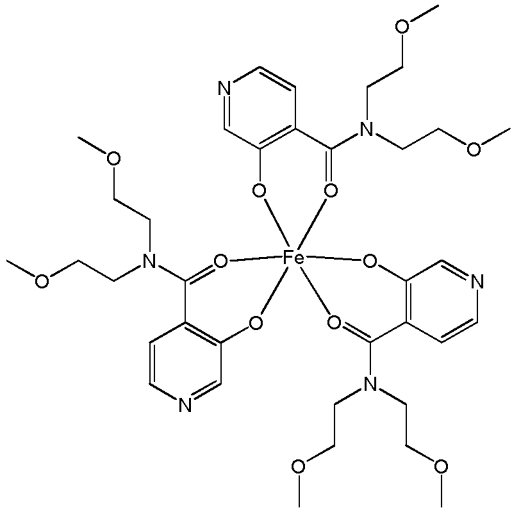

5.1 mmol (1.30 g) 3-Hydroxy-*N*,*N*-bis(2-methoxyethyl)isonicotinamid wurden in 50 ml Ethanol vorgelegt und 5.1 mmol Natriummethanolat (1.17 ml 25% Lösung in Methanol) zugegeben. Es wurden 1.7 mmol (0.277 g) $FeCl_3$ (wasserfrei) in 10 ml Ethanol gelöst zugetropft und 2 h gerührt. Das Reaktionsgemisch wurde filtriert, das Filtrat am Rotationsverdampfer eingeengt und der Rückstand bei 50°C im Vakuumtrockenschrank getrocknet. Man erhielt 1.53 g (98% Fe-Ausbeute) der Titelverbindung.
IR (in Substanz, $cm^{-1}$): 1631, 1556, 1469, 1429, 1397, 1364, 1309, 1221, 1203, 1181, 1140, 1111, 1067, 1015, 824, 791, 726, 701.
CHN-Elementaranalyse: C, 50.17; H, 6.30; N, 9.72.
Fe-Gehalt: 6.06% [m/m]
Chlorid-Gehalt: 0.76% [m/m]

**10. Tris-(*N*,*N*-Diethyl-3-hydroxyisonicotinamid)-eisen(III)-Komplex**

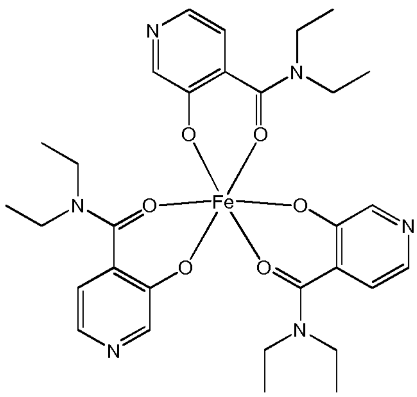

15 mmol (3.0 g) *N*,*N*-Diethyl-3-hydroxyisonicotinamid wurden in 100 ml Ethanol gelöst und 15 mmol Natriummethanolat (25% Lösung in Methanol) zugegeben. Es wurden 5 mmol (0.83 g) $FeCl_3$ (wasserfrei) in 10 ml Ethanol gelöst zugetropft und 2 h gerührt. Das Reaktionsgemisch wurde filtriert, das Filtrat am Rotationsverdampfer eingeengt und der Rückstand bei 50°C im Vakuumtrockenschrank getrocknet. Man erhielt 3.2 g (100% Fe-Ausbeute) der Titelverbindung.
IR (in Substanz, $cm^{-1}$): 1557, 1520, 1471, 1397, 1362, 1308, 1259, 1222, 1159, 1095, 1062, 1004, 947, 893, 824, 789, 725, 697, 645, 610.
CHN-Elementaranalyse: C, 53.48; H, 6.25; N, 12.33.
Fe-Gehalt: 8.72% [m/m]
Chlorid-Gehalt: 1.1% [m/m]

**11. Tris-((3-Hydroxypyridin-4-yl)(morpholino)methanon)-eisen(III)-Komplex**

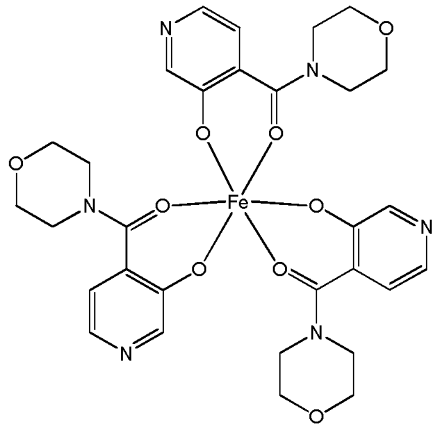

9 mmol (1.93 g) (3-Hydroxypyridin-4-yl)(morpholino)methanon wurden in 50 ml Ethanol vorgelegt und 9 mmol Natriummethanolat (25% Lösung in Methanol) zugegeben. Es wurden 3 mmol (0.497 g) $FeCl_3$ (wasserfrei) in 10 ml Ethanol gelöst zugetropft und 2 h gerührt. Das Reaktionsgemisch wurde filtriert, das Filtrat am Rotationsverdampfer eingeengt und der Rückstand bei 50°C im Vakuumtrockenschrank getrocknet. Man erhielt 2.2 g (98% Fe-Ausbeute) der Titelverbindung.
IR (in Substanz, $cm^{-1}$): 1624, 1559, 1523, 1469, 1435, 1398, 1361, 1305, 1281, 1266, 1219, 1181, 1137, 1110, 1063, 1022, 939, 902, 865, 827, 788, 724, 705, 626. CHN-Elementaranalyse: C, 50.36; H, 5.52; N, 11.48.
Fe-Gehalt: 7.43% [m/m]
Chlorid-Gehalt: 0.67% [m/m]

**12. Tris-((3-Hydroxypyridin-4-yl)(piperidin-1-yl)methanon)-eisen(III)-Komplex**

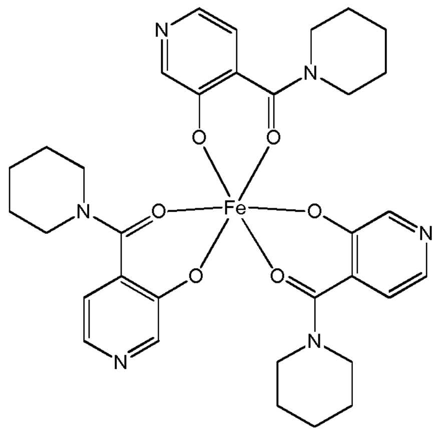

15 mmol (3.12 g) (3-Hydroxypyridin-4-yl)(piperidin-1-yl)methanon wurden in 80 ml Ethanol vorgelegt und 15 mmol Natriummethanolat (25% Lösung in Methanol) zugegeben. Es wurden 5 mmol (0.83 g) $FeCl_3$ (wasserfrei) in 10 ml Ethanol gelöst zugetropft und 2 h gerührt. Das Reaktionsgemisch wurde filtriert, das Filtrat am Rotationsverdampfer eingeengt und der Rückstand bei 50°C im Vakuumtrockenschrank getrocknet. Man erhielt 3.3 g (98% Fe-Ausbeute) der Titelverbindung.
IR (in Substanz, $cm^{-1}$): 1556, 1524, 1469, 1442, 1397, 1309, 1285, 1258, 1235, 1216, 1187, 1149, 1105, 1062, 1025, 1004, 954, 927, 894, 853, 822, 788, 724, 701, 646, 616. CHN-Elementaranalyse: C, 55.39; H, 6.024; N, 11.48.
Fe-Gehalt: 8.27% [m/m]
Chlorid-Gehalt: 0.88% [m/m]

**13. Tris-((3-Hydroxypyridin-4-yl)(pyrrolidin-1-yl)methanon)-eisen(III)-Komplex**

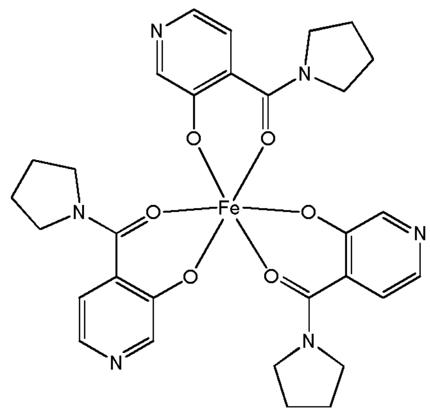

9 mmol (1.73 g) (3-Hydroxypyridin-4-yl)(pyrrolidin-1-yl)methanon wurden in 70 ml Ethanol vorgelegt und 9 mmol Natriummethanolat (2.06 ml 25% Lösung in Methanol) zugegeben. Es wurden 3 mmol (0.497 g) $FeCl_3$ (wasserfrei) in 10 ml Ethanol gelöst zugetropft und 2 h gerührt. Das Reaktionsgemisch wurde filtriert, das Filtrat am Rotationsverdampfer eingeengt und der Rückstand bei 50°C im Vakuumtrockenschrank getrocknet. Man erhielt 1.98 g (94% Fe-Ausbeute) der Titelverbindung.

IR (in Substanz, $cm^{-1}$): 1552, 1517, 1452, 1393, 1310, 1272, 1223, 1205, 1171, 1128, 1065, 914, 871, 827, 787, 741, 712, 657.

CHN-Elementaranalyse: C, 53.47; H, 5.49; N, 12.26.

Fe-Gehalt: 7.98% [m/m]

Chlorid-Gehalt: 1.85% [m/m]

## TESTMETHODE:

Die hervorragenden Fe-Utilisationen, die durch die erfindungsgemäßen Fe-Komplexe, erreicht werden können, wurden durch folgendes Mäuse-Modell gemessen.

Männliche NMRI (SPF)-Mäuse (ca. 3 Wochen alt) wurden ca. 3 Wochen mit eisenarmer Diät (ca. 5 ppm Eisen) gefüttert. Dann wurde jeweils 6 Mäusen während 2 mal 5 Tagen mit einer Unterbrechung von 2 Tagen (Tage 1 - 5 und 8 - 12) die Eisenkomplexe mittels Schlundsonde verabreicht (2 mg Eisen/kg Körpergewicht/Tag). 6 Mäuse dienten als Kontrollgruppe (Negativkontrolle) und erhielten stattdessen Wasser. Die Utilisation am Tag 15 wurde berechnet aus der Hämoglobinzunahme und der Körpergewichtszunahme nach der Formel

$$\text{Utilisation (\%)} = \frac{\Delta \text{Eisenutilisation} * 100}{\text{Fe Dos.}} = \frac{(\text{Fe ut.} - \text{Fe ut.Control}) * 100}{\text{Fe Dos.}}$$

$$= [(Hb_{2(3)} * BW_{9(14)} - Hb_1 * BW_4) * 0{,}07 * 0{,}0034 - (Hb_{2(3)\,Control} * BW_{9(14)\,Control} - Hb_{1\,Control} * BW_{4\,Control}) * 0{,}07 * 0{,}0034)] * 100/\text{ Fe Dos.}$$

$$= [(Hb_{2(3)} * BW_{9(14)} - Hb_1 * BW_4) * 0{,}000238 - (Hb_{2(3)\,Control} * BW_{9(14)\,Control} - Hb_{1\,Control} * BW_{4\,Control}) * 0{,}000238] * 100 / \text{Fe Dos.}$$

$$= (Hb_{2(3)} * BW_{9(14)} - Hb_1 * BW_4 - Hb_{2(3)\,Control} * BW_{9(14)\,Control} + Hb_{1\,Control} * BW_{4\,Control}) * 0{,}0238 / \text{Fe Dos.}$$

| | |
|---|---|
| 0.07 | = Faktor für 70 ml Blut per kg Körpergewicht (BW) |
| 0.0034 | = Faktor für 0,0034 g Fe/g Hb |
| $Hb_1$ | = Hämoglobin-Wert (g/l) am Tag 1 |
| $Hb_{2(3)}$ | = Hämoglobin-Wert (g/l) am Tag 8 (oder 15) |
| $BW_4$ | = Körpergewicht (g) am Tag 1 |
| $BW_{9(14)}$ | = Körpergewicht (g) am Tag 8 (oder 15) |
| $Hb_{1\,Control}$ | = Durchschnitts-Hämoglobinwert (g/l) am Tag 1 in der Kontrollgruppe, |
| $Hb_{2(3)\,Control}$ | = Durchschnitts-Hämoglobinwert (g/l) am Tag 8 (oder 15) in der Kontrollgruppe, |
| $BW_{4\,Control}$ | = Durchschnittskörpergewicht (g) am Tag 1 in der Kontrollgruppe, |
| $BW_{9(14)\,Control}$ | = Durchschnittskörpergewicht (g) am Tag 8 (oder 15) in der Kontrollgruppe, |
| Fe Dos. | = Gesamtes verabreichtes Eisen (mg Fe) während 5 oder 10 Tage, |
| Fe ut. | = $(Hb_{2(3)} * BW_{9(14)} - Hb_1 * BW_4) * 0.07 * 0.0034$ (mg Fe) |
| Δ Utilisation | = Fe tot. utilisiert (Untersuchte Gruppe) - Fe ut. Kontrollgruppe, utilisiert aus Nahrung, (mg Fe) |

Die nachfolgende Tabelle 1 zeigt die Eisenutilisationen:

Tabelle 1:

| Beispiel-Nr. | Utilisation n 15 d (abs. %) | Standardabweichung (+/- 0,5) |
|---|---|---|
| 1 | 68 | 11 |
| 6 | 76 | 10 |
| 7 | 76 | 10 |

Vergleichsbeispiele:

Zum Vergleich wurden die aus der WO2011117225 beschriebenen Liganden AAO. N,N-Dimethyl-2-oxocyclohexancarboxamid

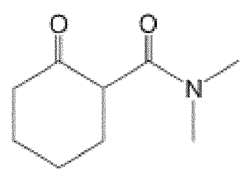

und

AAP. N,N-Dimethyl-2-oxocyclopentancarboxamid

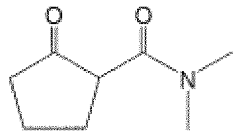

sowie die Komplex-Verbindung nach Beispiel 66
Tris-(N,N-Dimethyl-2-oxocyclopentancarboxamid)-eisen(III)-Komplex

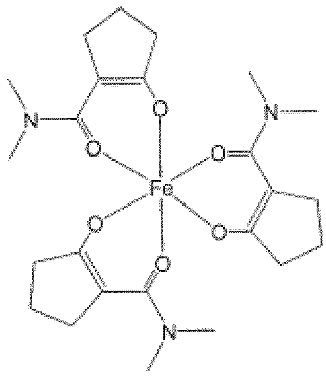

hergestellt.

Der Komplex nach Beispiel 66 der WO 2011117225 zeigte eine sehr geringe Stabilität. Der Eisen-Komplex konnte zwar dargestellt und in vitro getestet werden, es konnte aber aufgrund seiner geringen Stabilität keine Formulierung gefunden werden, die erlaubt hätte ihn auch in vivo im vorstehend beschriebenen Mausmodell zu testen.

Der Ligand mit der analogen 6-Ring-Struktur (Ligand AAO) zeigte eine noch geringere Stabilität. Hier konnte zwar der Ligand dargestellt werden, der Komplex hingegen war aber so instabil das es nicht einmal möglich war diesen rein darzustellen und analytisch zu vermessen, geschweige denn in vitro oder in vivo zu testen.

Aufgrund ihrer sehr geringen Stabilitäten sind derartige Verbindungen daher für medizinische Anwendungen als völlig ungeeignet anzusehen.

Überraschenderweise konnten hingegen durch die neuen Komplexe der vorliegenden Erfindung diese Probleme überwunden werden. Es konnte in vivo gezeigt werden, dass diese neuen Komplexe eine ausreichende Stabilität besitzen und sich sehr gut für eine medizinische Anwendung in der Eisentherapie einsetzen lassen. Die Testungen im Mausmodell lieferten hervorragende Utilisationsdaten.

Die gemessenen Eisen-Utilisationswerte stellen einen wichtigen Parameter im Hinblick auf die Indikation der Behandlung von Eisenmangelerscheinungen und Eisenmangelanämien dar, denn dieser Parameter spiegelt nicht nur die Eisenaufnahme, sondern auch den Zusammenhang zwischen Körpergewicht und Eisenaufnahme wieder, was gerade bei der Verwendung von heranwachsenden Tieren im Tiermodell wichtig ist. Würde man nur die Hämoglobin-Werte betrachten, die ein Maß für das wirklich aufgenommene und verwendete Eisen darstellen, würde man den auf dem Wachstum der Tiere beruhenden Anteil unberücksichtigt lassen. Somit stellt die EisenUtilisation eine genauere Meßgröße dar, obwohl Eisenutilisation und Hämoglobinwerte meist miteinander korrelieren. Eine Betrachtung des reinen Eisen-Serumspiegels, welche ebenfalls messbar wäre, kommt weniger in Betracht, da zwar eine Aussage über die Menge Eisen getroffen wird, die in den Körper gelangt, aber nicht darüber, wieviel der Körper davon auch nutzen kann.

Die Testergebnisse zeigen, dass die erfindungsgemäßen Eisenkomplexverbindungen über eine hervorragende Eisenutilisation verfügen, so dass sie als Mittel zur Behandlung von Eisenmangelanämien und der damit verbundenen Symptome geeignet sind.

## BEVORZUGTE AUSFÜHRUNGSFORMEN:

Weitere bevorzugte Ausführungsformen der vorliegenden Erfindung betreffen:

1. Eisen(III)-3-hydroxyisonicotinamid-Komplexverbindungen oder pharmazeutische verträgliche Salze davon zur Anwendung in der Behandlung und Prophylaxe von Eisenmangelerscheinungen und Eisenmangelanämien, enthaltend mindestens einen Liganden der Formel (I):

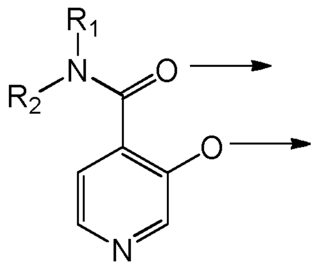

(I)

worin
die Pfeile jeweils eine koordinative Bindung zu einem oder verschiedenen Eisenatomen darstellen,
$R_1$ und $R_2$ gleich oder verschieden sind, und jeweils ausgewählt werden aus der Gruppe, die besteht aus

- Wasserstoff, und
- gegebenenfalls substituiertem Alkyl,

oder pharmazeutisch verträgliche Salze davon.

2. Eisen(III)-Komplexverbindungen zur Anwendung nach Ausführungsform 1, enthaltend mindestens einen Liganden der Formel (I):

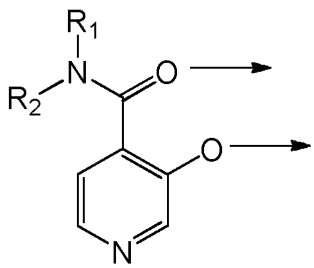

(I)

worin
die Pfeile jeweils eine koordinative Bindung zu einem oder verschiedenen Eisenatomen darstellen,
$R_1$ und $R_2$ gleich oder verschieden sind, und jeweils ausgewählt werden aus der Gruppe, die besteht aus

- Wasserstoff, und
- gegebenenfalls mit einer Alkoxy oder Hydroxy-Gruppe, bevorzugt mit einer Alkoxy-Gruppe, substituiertem Alkyl, umfassend auch Alkyl, worin eine oder zwei Methylengruppen ($-CH_2-$) durch -O- ersetzt sein können,

oder pharmazeutisch verträgliche Salze davon.

3. Eisen(III)-Komplexverbindungen zur Anwendung nach einer der Ausführungsformen 1 oder 2, enthaltend mindestens einen Liganden der Formel (I):

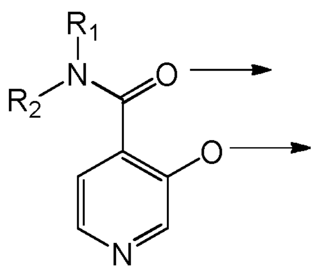

(I)

worin
die Pfeile jeweils eine koordinative Bindung zu einem oder verschiedenen Eisenatomen darstellen,
$R_1$ und $R_2$ gleich oder verschieden sind, und jeweils ausgewählt werden aus der Gruppe, die besteht aus: Wasserstoff, Methyl, Ethyl, Propyl, Isopropyl, n-Butyl, sek-Butyl Isobutyl, Methoxyethyl, Ethoxyethyl, Methoxypropyl und Ethoxypropyl, Methoxyethoxyethyl, Ethoxyethoxyethyl, Propoxyethoxyethyl; oder pharmazeutisch verträgliche Salze davon.

4. Eisen(III)-Komplexverbindungen zur Anwendung nach einer der Ausführungsformen 1 bis 3, enthaltend mindestens einen Liganden der Formel (I)

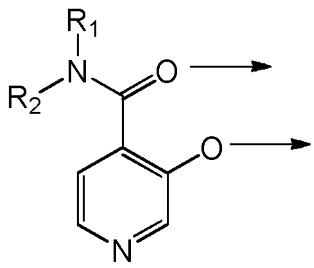

(I)

worin
die Pfeile jeweils eine koordinative Bindung zu einem oder verschiedenen Eisenatomen darstellen,
$R_1$ und $R_2$ gleich oder verschieden sind, und jeweils ausgewählt werden aus der Gruppe, die besteht aus gegebenenfalls substituiertem Alkyl, vorzugsweise aus Methyl und Ethyl,
oder pharmazeutisch verträgliche Salze davon.

5. Eisen(III)-Komplexverbindungen zur Anwendung nach einer der Ausführungsformen 1 bis 4 der Formel (II):

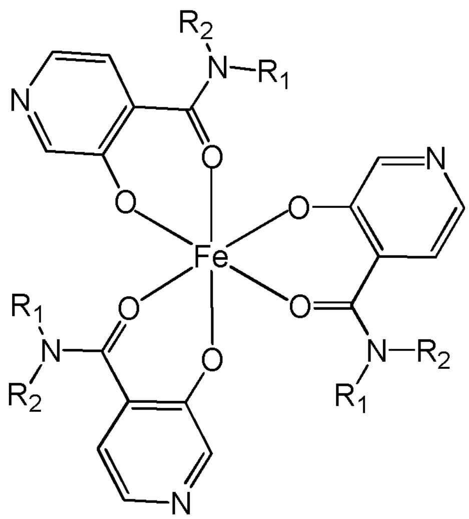

(II)

worin $R_1$ und $R_2$ wie oben definiert sind
oder pharmazeutisch verträgliche Salze davon.

6. Eisen(III)-Komplexverbindungen zur Anwendung nach einer der Ausführungsformen 1 bis 5 zur Behandlung und Prophylaxe der Symptome von Eisenmangelerscheinungen und Eisenmangelanämien.

7. Eisen(III)-Komplexverbindungen zur Anwendung nach Ausführungsform 6, worin die Symptome einschließen: Ermüdung, Antriebslosigkeit, Konzentrationsschwäche, geringe kognitive Effizienz, Schwierigkeiten beim Finden der richtige Worte, Vergesslichkeit, unnatürliche Blässe, Reizbarkeit, Beschleunigung der Herzfrequenz (Tachykardie), wunde oder geschwollene Zunge, vergrößerte Milz, Schwangerengelüste (Pica), Kopfschmerzen, Appetitlosigkeit, erhöhte Infektionsanfälligkeit, depressive Verstimmungen.

8. Eisen(III)-Komplexverbindungen zur Anwendung nach einer der Ausführungsformen 1 bis 7 zur Behandlung und Prophylaxe der Eisenmangelanämie bei Schwangeren, der latenten Eisenmangelanämie bei Kindern und Heranwachsenden, der Eisenmangelanämie infolge gastrointestinaler Abnormalitäten, der Eisenmangelanämie infolge

von Blutverlusten, wie durch gastrointestinale Blutungen (z.B. infolge von Geschwüren, Karzinomen, Hämorriden, entzündlichen Störungen, Einnahme von Acetylsalicylsäure), der Eisenmangelanämie infolge von Menstruation, der Eisenmangelanämie infolge von Verletzungen, der Eisenmangelanämie infolge von Psilosis (sprue), der Eisenmangelanämie infolge verminderter Eisenaufnahme bei der Ernährung, insbesondere bei selektiv essenden Kindern und Heranwachsenden, der Immunschwäche hervorgerufen durch Eisenmangelanämie, der Beeinträchtigung der Hirnleistung hervorgerufen durch Eisenmangelanämien, des Restless Leg Syndrom hervorgerufen durch Eisenmangelanämien, Eisenmangelanämien bei Krebs, Eisenmangelanämien ausgelöst durch Chemotherapien, Eisenmangelanämien ausgelöst durch Inflammation (AI), Eisenmangelanämien bei kongestiver Herzinsuffizienz (CHF;congestive heart failure), Eisenmangelanämien bei chronischer Niereninsuffizienz Stadium 3-5 (CKD 3-5; chronic kidney diseases stage 3-5), Eisenmangelanämien ausgelöst durch chronische Inflammation (ACD), Eisenmangelanämien bei rheumatischer Arthritis (RA; rheumatoid arthritis), Eisenmangelanämien bei systemischem Lupus erythemotodes (SLE; systemic lupus erythematosus) und Eisenmangelanämien bei inflammatorischen Darmerkrankungen (IBD; inflammatory bowel diseases).

9. Eisen(III)-Komplexverbindungen zur Anwendung nach einer der Ausführungsformen 1 bis 8 zur oralen Verabreichung.

10. Eisen(III)-Komplexverbindungen nach Ausführungsform 9 zur Verabreichung als Tablette oder Kapsel.

11. Arzneimittel, enthaltend Eisen(III)-Komplexverbindungen wie in einer der Ausführungsformen 1 bis 5 definiert.

12. Arzneimittel, enthaltend Eisen(III)-Komplexverbindungen wie in einer der Ausführungsformen 1 bis 5 definiert sowie mindestens einen physiologisch verträglichen Träger bzw. Exzipienten.

13. Zusammensetzungen, die Eisen(III)-Komplexverbindungen enthalten, wie in einer der Ausführungsformen 1 bis 5 definiert, in Kombination mit mindestens einem weiteren Arzneimittel welches auf den Eisenmetabolismus wirkt.

## Patentansprüche

1. Eisen(III)-3-hydroxyisonicotinamid-Komplexverbindungen oder pharmazeutische verträgliche Salze davon zur Anwendung in der Behandlung und Prophylaxe von Eisenmangelerscheinungen und Eisenmangelanämien.

2. Eisen(III)-Komplexverbindungen zur Anwendung nach Anspruch 1, enthaltend mindestens einen Liganden der Formel (I):

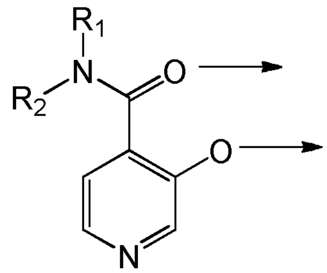

(I)

worin
die Pfeile jeweils eine koordinative Bindung zu einem oder verschiedenen Eisenatomdarstellen ;
$R_1$ und $R_2$ gleich oder verschieden sind, und jeweils ausgewählt werden aus der Gruppe, die besteht aus:

- Wasserstoff und
- gegebenenfalls substituiertem Alkyl, oder worin

$R_1$ und $R_2$ gemeinsam mit dem Stickstoffatom, an welches sie gebunden sind, einen gegebenenfalls substituierten 3- bis 6-gliedrigen Ring bilden, der gegebenenfalls ein weiteres Heteroatom aufweisen kann;
oder pharmazeutisch verträgliche Salze davon.

**3.** Eisen(III)-Komplexverbindungen zur Anwendung nach Anspruch 1 oder 2, enthaltend mindestens einen Liganden der Formel (I):

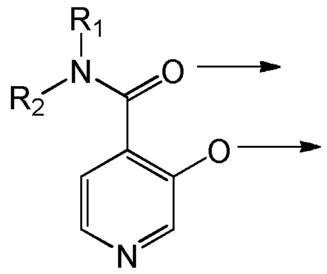

(I)

worin
die Pfeile jeweils eine koordinative Bindung zu einem oder verschiedenen Eisenatomen darstellen,
$R_1$ und $R_2$ gleich oder verschieden sind, und jeweils ausgewählt werden aus der Gruppe, die besteht aus

- Wasserstoff, und
- gegebenenfalls substituiertem Alkyl, oder worin

$R_1$ und $R_2$ gemeinsam mit dem Stickstoffatom, an welches sie gebunden sind, einen gegebenenfalls substituierten 5- bis 6-gliedrigen Ring bilden, der gegebenenfalls ein weiteres Heteroatom aufweisen kann;
oder pharmazeutisch verträgliche Salze davon.

**4.** Eisen(III)-Komplexverbindungen zur Anwendung nach einem der Ansprüche 1 bis 3, enthaltend mindestens einen Liganden der Formel (I):

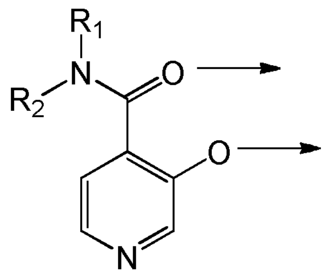

(I)

worin
die Pfeile jeweils eine koordinative Bindung zu einem oder verschiedenen Eisenatomen darstellen,
$R_1$ und $R_2$ gleich oder verschieden sind, und jeweils ausgewählt werden aus der Gruppe, die besteht aus

- Wasserstoff, und
- gegebenenfalls mit einer Alkoxy oder Hydroxy-Gruppe, bevorzugt mit einer Alkoxy-Gruppe, substituiertem Alkyl, umfassend auch Alkyl, worin eine oder zwei Methylengruppen ($-CH_2-$) durch -O- ersetzt sein können, oder worin

$R_1$ und $R_2$ gemeinsam mit dem Stickstoffatom, an welches sie gebunden sind, einen gegebenenfalls substituierten 5- bis 6-gliedrigen Ring bilden, der gegebenenfalls ein weiteres Heteroatom aufweisen kann;
oder pharmazeutisch verträgliche Salze davon.

**5.** Eisen(III)-Komplexverbindungen zur Anwendung nach einem der Ansprüche 1 bis 4, enthaltend mindestens einen Liganden der Formel (I):

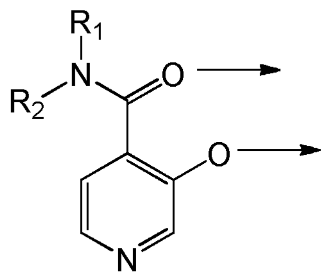

(I)

worin
die Pfeile jeweils eine koordinative Bindung zu einem oder verschiedenen Eisenatomen darstellen,
$R_1$ und $R_2$ gleich oder verschieden sind, und jeweils ausgewählt werden aus der Gruppe, die besteht aus: Wasserstoff, Methyl, Ethyl, Propyl, Isopropyl, n-Butyl, sek-Butyl, Isobutyl, Methoxyethyl, Ethoxyethyl, Methoxypropyl und Ethoxypropyl, Methoxyethoxyethyl, Ethoxyethoxyethyl, Propoxyethoxyethyl; oder worin
$R_1$ und $R_2$ gemeinsam mit dem Stickstoffatom, an welches sie gebunden sind, einen heterocyclischen 5- bis 6-gliedrigen Ring bilden, der ausgewählt ist aus der Gruppe bestehend aus Morpholinyl, Piperidinyl und Pyrrolidinyl; oder pharmazeutisch verträgliche Salze davon.

**6.** Eisen(III)-Komplexverbindungen zur Anwendung nach einem der Ansprüche 1 bis 5, enthaltend mindestens einen Liganden der Formel (I)

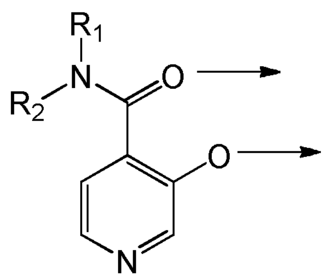

(I)

worin
die Pfeile jeweils eine koordinative Bindung zu einem oder verschiedenen Eisenatomen darstellen,
$R_1$ und $R_2$ gleich oder verschieden sind, und jeweils ausgewählt werden aus der Gruppe, die besteht aus Wasserstoff, gegebenenfalls substituiertem Alkyl, ausgewählt aus der Gruppe, die besteht aus Methyl, Ethyl, Propyl, n-Butyl und mit einer Methoxy-Gruppe oder einer Ethoxy-Gruppe, substituiertem Alkyl; oder worin
$R_1$ und $R_2$ gemeinsam mit dem Stickstoffatom, an welches sie gebunden sind, einen heterocyclischen 5- bis 6-gliedrigen Ring bilden, der ausgewählt ist aus der Gruppe bestehend aus Morpholinyl, Piperidinyl und Pyrrolidinyl oder pharmazeutisch verträgliche Salze davon.

**7.** Eisen(III)-Komplexverbindungen zur Anwendung nach einem der Ansprüche 1 bis 6 der Formel (II):

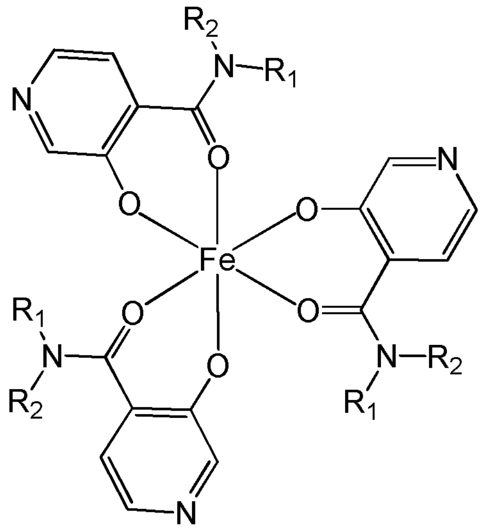

(II)

worin $R_1$ und $R_2$ wie in einem der vorstehenden Ansprüche definiert sind oder pharmazeutisch verträgliche Salze davon.

**8.** Eisen(III)-Komplexverbindungen zur Anwendung nach einem der Ansprüche 1 bis 7, die ausgewählt sind aus der Gruppe:

Bsp. 1

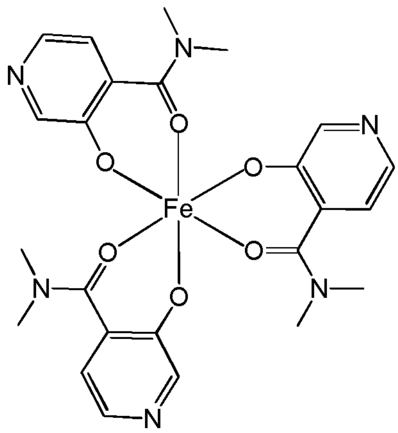

,

Bsp. 2

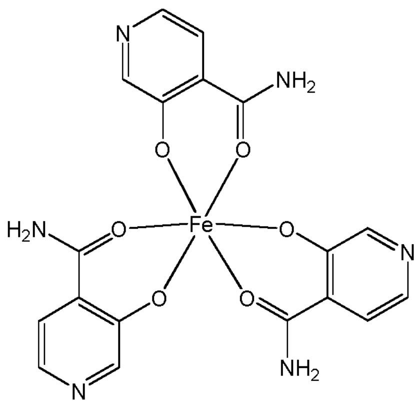

,

Bsp. 3

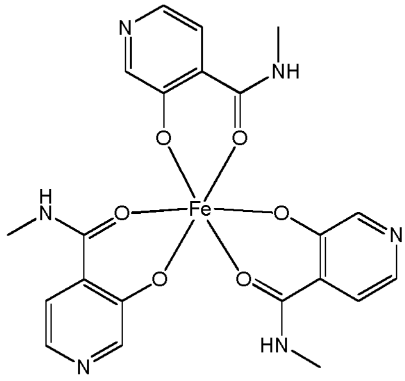

,

Bsp. 4

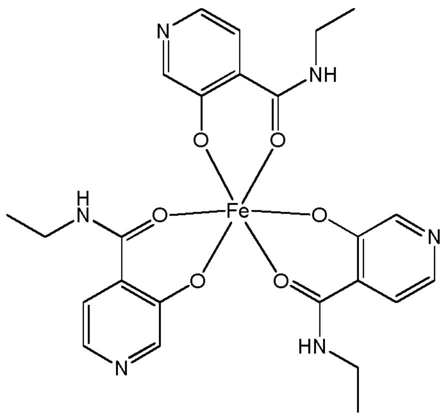

,

Bsp. 5

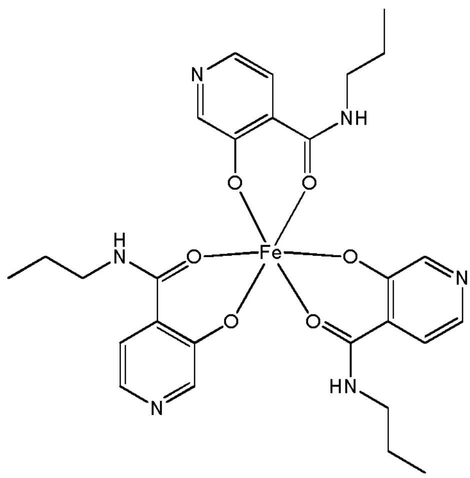

,

Bsp. 6

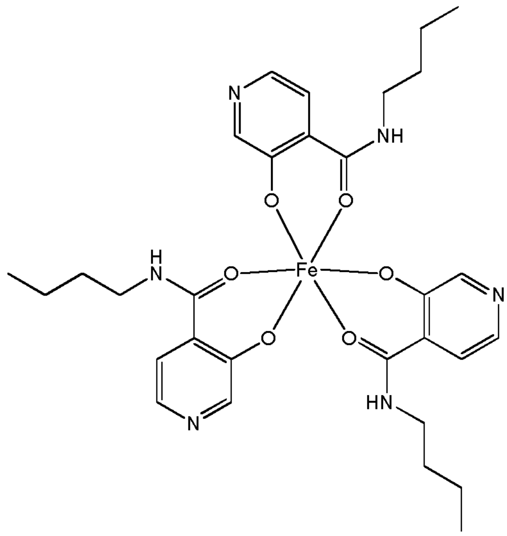

,

Bsp. 7

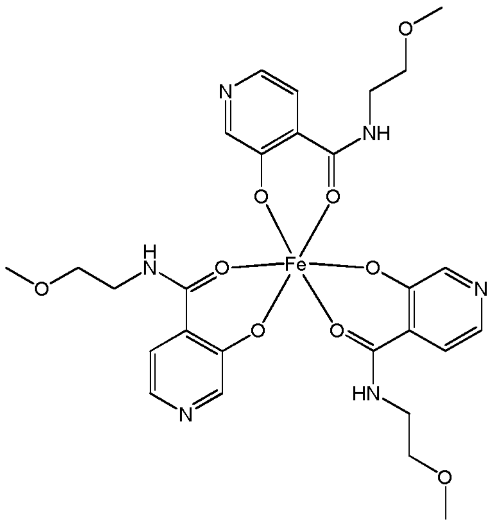

,

Bsp. 8

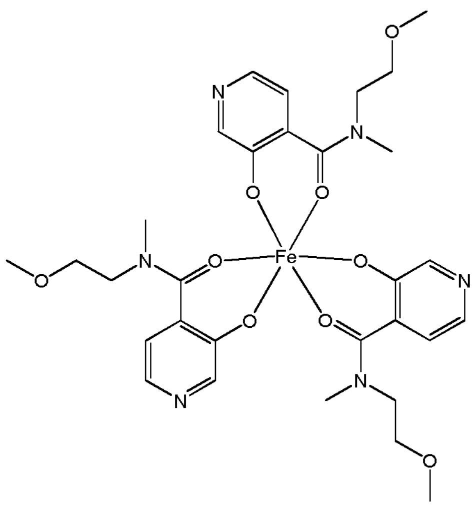

,

Bsp. 9

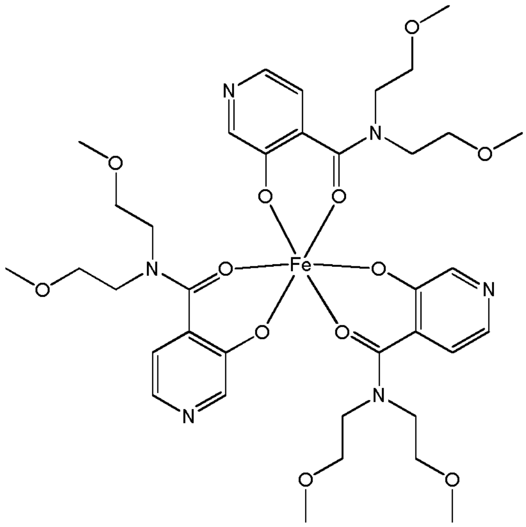

,

Bsp. 10

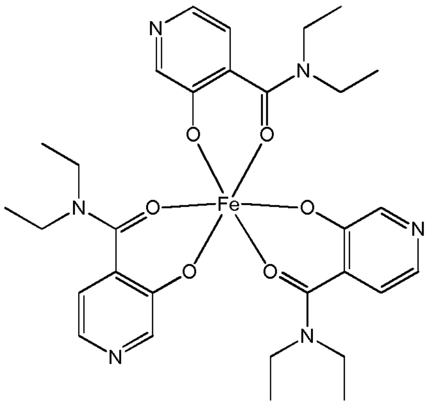

,

Bsp. 11

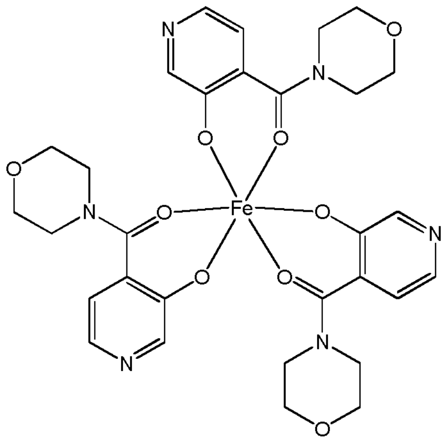

,

Bsp. 12

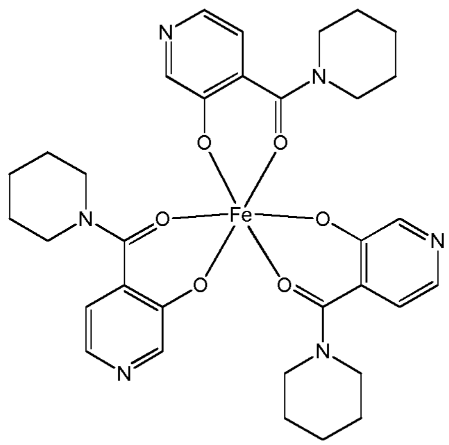

und
Bsp. 13

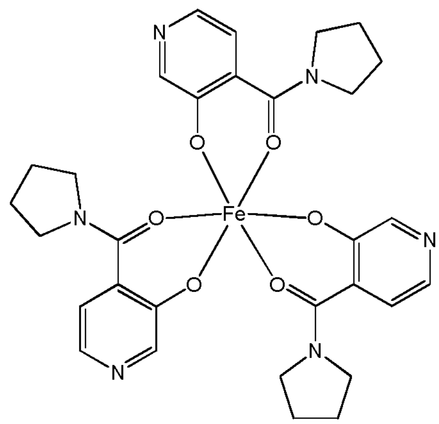

9. Eisen(III)-Komplexverbindungen zur Anwendung nach einem der Ansprüche 1 bis 8 zur Behandlung und Prophylaxe der Symptome von Eisenmangelerscheinungen und Eisenmangelanämien.

10. Eisen(III)-Komplexverbindungen zur Anwendung nach Anspruch 9, worin die Symptome einschließen: Ermüdung, Antriebslosigkeit, Konzentrationsschwäche, geringe kognitive Effizienz, Schwierigkeiten beim Finden der richtige Worte, Vergesslichkeit, unnatürliche Blässe, Reizbarkeit, Beschleunigung der Herzfrequenz (Tachykardie), wunde oder geschwollene Zunge, vergrößerte Milz, Schwangerengelüste (Pica), Kopfschmerzen, Appetitlosigkeit, erhöhte Infektionsanfälligkeit, depressive Verstimmungen.

11. Eisen(III)-Komplexverbindungen zur Anwendung nach einem der Ansprüche 1 bis 10 zur Behandlung und Prophylaxe der Eisenmangelanämie bei Schwangeren, der latenten Eisenmangelanämie bei Kindern und Heranwachsenden, der Eisenmangelanämie infolge gastrointestinaler Abnormalitäten, der Eisenmangelanämie infolge von Blutverlusten, wie durch gastrointestinale Blutungen (z.B. infolge von Geschwüren, Karzinomen, Hämorriden, entzündlichen Störungen, Einnahme von Acetylsalicylsäure), der Eisenmangelanämie infolge von Menstruation, der Eisenmangelanämie infolge von Verletzungen, der Eisenmangelanämie infolge von Psilosis (sprue), der Eisenmangelanämie infolge verminderter Eisenaufnahme bei der Ernährung, insbesondere bei selektiv essenden Kindern und Heranwachsenden, der Immunschwäche hervorgerufen durch Eisenmangelanämie, der Beeinträchtigung der Hirnleistung hervorgerufen durch Eisenmangelanämien, des Restless Leg Syndrom hervorgerufen durch Eisenmangelanämien, Eisenmangelanämien bei Krebs, Eisenmangelanämien ausgelöst durch Chemotherapien, Eisenmangelanämien ausgelöst durch Inflammation (AI), Eisenmangelanämien bei kongestiver Herzinsuffizienz (CHF;congestive heart failure), Eisenmangelanämien bei chronischer Niereninsuffizienz Stadium 3-5 (CKD 3-5; chronic kidney diseases stage 3-5), Eisenmangelanämien ausgelöst durch chronische Inflammation (ACD), Eisenmangelanämien bei rheumatischer Arthritis (RA; rheumatoid arthritis), Eisenmangelanämien bei systemischem Lupus erythemotodes (SLE; systemic lupus erythematosus) und Eisenmangelanämien bei inflammatorischen Darmerkrankungen (IBD;

inflammatory bowel diseases).

**12.** Eisen(III)-Komplexverbindungen zur Anwendung nach einem der Ansprüche 1 bis 11 zur oralen Verabreichung.

**13.** Eisen(III)-Komplexverbindungen zur Anwendung nach Anspruch 12 zur Verabreichung als Tablette oder Kapsel.

**14.** Arzneimittel, enthaltend Eisen(III)-Komplexverbindungen wie in einem der Ansprüche 1 bis 8 definiert.

**15.** Arzneimittel, enthaltend Eisen(III)-Komplexverbindungen wie in einem der Ansprüche 1 bis 8definiert sowie mindestens einen physiologisch verträglichen Träger bzw. Exzipienten.

**16.** Zusammensetzungen, die Eisen(III)-Komplexverbindungen enthalten, wie in einem der Ansprüche 1 bis 8 definiert, in Kombination mit mindestens einem weiteren Arzneimittel welches auf den Eisenmetabolismus wirkt.

**Claims**

**1.** Iron(III)-3-hydroxyisonicotinamide complex compounds or pharmaceutically acceptable salts thereof for the use in the treatment and prophylaxis of iron deficiency symptoms and iron deficiency anemias.

**2.** Iron(III) complex compounds for the use according to claim 1, containing at least one ligand of the formula (I):

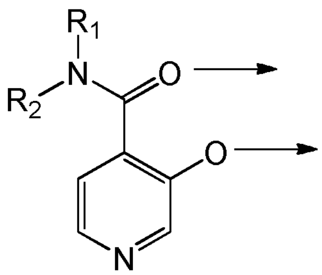

(I)

wherein
the arrows respectively represent a coordinate bond to one or different iron atoms;
$R_1$ and $R_2$ are the same or different and are each selected from the group consisting of:

- hydrogen and
- optionally substituted alkyl, or wherein

$R_1$ and $R_2$ together with the nitrogen atom to which they are bonded form an optionally substituted 3- to 6-membered ring, which may optionally contain a further heteroatom;
or pharmaceutically acceptable salts thereof.

**3.** Iron(III) complex compounds for the use according to claim 1 or 2, containing at least one ligand of the formula (I):

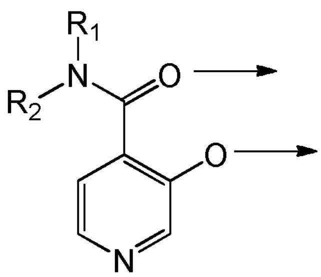

(I)

wherein

the arrows respectively represent a coordinate bond to one or different iron atoms,
$R_1$ and $R_2$ are the same or different and are each selected from the group consisting of:

- hydrogen, and
- optionally substituted alkyl, or wherein

$R_1$ and $R_2$ together with the nitrogen atom to which they are bonded form an optionally substituted 5- to 6-membered ring, which may optionally contain a further heteroatom;
or pharmaceutically acceptable salts thereof.

**4.** Iron(III) complex compounds for the use according to one of the claims 1 through 3, containing at least one ligand of the formula (I):

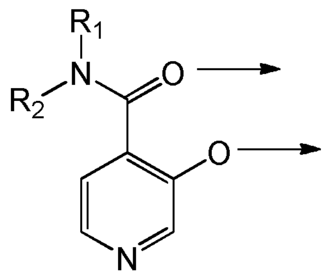

(I)

wherein
the arrows respectively represent a coordinate bond to one or different iron atoms,
$R_1$ and $R_2$ are the same or different and are each selected from the group consisting of:

- hydrogen, and
- alkyl, which may be substituted with an alkoxy or a hydroxy group, preferably with an alkoxy group, also comprising alkyl, wherein one or two methylene groups ($-CH_2-$) may be replaced by -O-, or wherein

$R_1$ and $R_2$ together with the nitrogen atom to which they are bonded form an optionally substituted 5- to 6-membered ring, which may optionally contain a further heteroatom;
or pharmaceutically acceptable salts thereof.

**5.** Iron(III) complex compounds for the use according to one of the claims 1 through 4, containing at least one ligand of the formula (I):

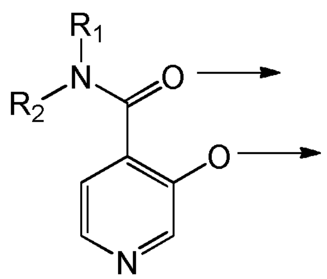

(I)

wherein
the arrows respectively represent a coordinate bond to one or different iron atoms,
$R_1$ and $R_2$ are the same or different and are each selected from the group consisting of: hydrogen, methyl, ethyl, propyl, isopropyl, n-butyl, sek-butyl isobutyl, methoxyethyl, ethoxyethyl, methoxypropyl and ethoxypropyl, methoxyethoxyethyl, ethoxyethoxyethyl, propoxyethoxyethyl; or wherein
$R_1$ and $R_2$ together with the nitrogen atom to which they are bonded form an optionally substituted 5- to 6-membered ring, which is selected from the group consisting of morpholinyl, piperidinyl and pyrrolidinyl;
or pharmaceutically acceptable salts thereof.

6. Iron(III) complex compounds for the use according to one of the claims 1 through 5, containing at least one ligand of the formula (I):

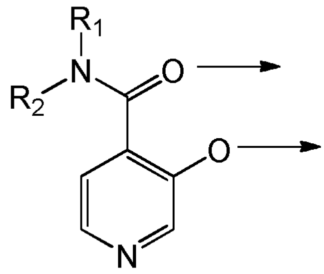

(I)

wherein
the arrows respectively represent a coordinate bond to one or different iron atoms,
$R_1$ and $R_2$ are the same of different and are each selected from the group consisting of hydrogen, optionally substituted alkyl, selected from the group consisting of methyl, ethyl, propyl, n-butyl and alkyl which is substituted with a methoxy group or an ethoxy group; or wherein
$R_1$ and $R_2$ together with the nitrogen atom to which they are bonded form a heterocyclic 5- to 6-membered ring, which is selected from the group consisting of morpholinyl, piperidinyl and pyrrolidinyl;
or pharmaceutically acceptable salts thereof.

7. Iron(III) complex compounds for the use according to one of the claims 1 through 6 of the formula (II):

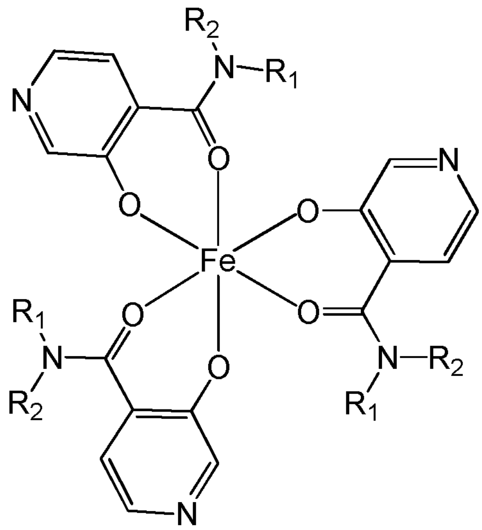

(II)

wherein $R_1$ and $R_2$ are as defined in any of the previous claims
or pharmaceutically acceptable salts thereof.

8. Iron(III) complex compounds for the use according to one of the claims 1 through 7, which are selected from the group:

| | |
|---|---|
| Exp. 1 | 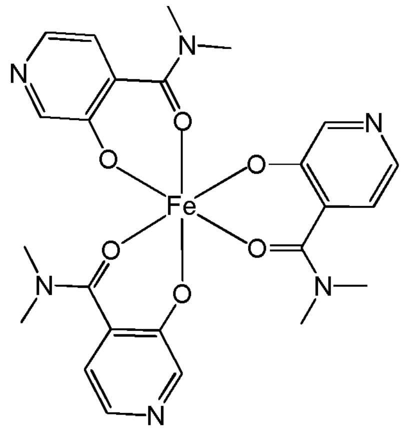 , |
| Exp. 2 | 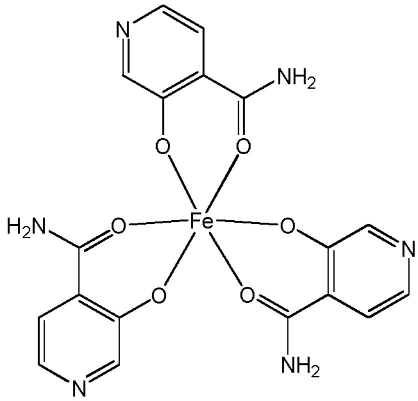 , |
| Exp. 3 | 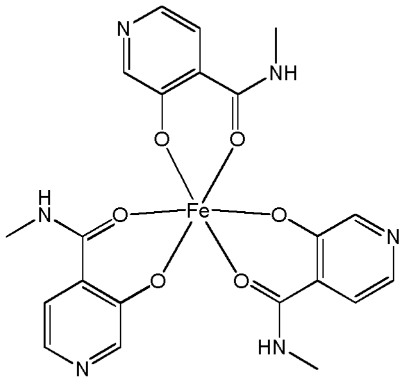 , |
| Exp. 4 | 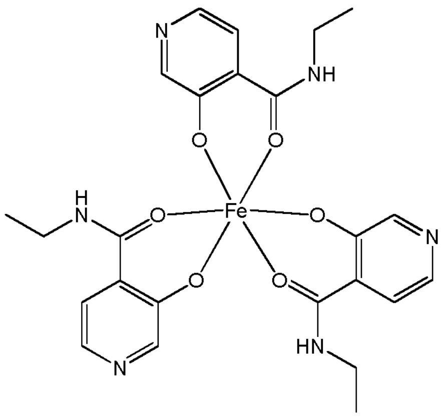 , |

(continued)

| | |
|---|---|
| Exp. 5 | 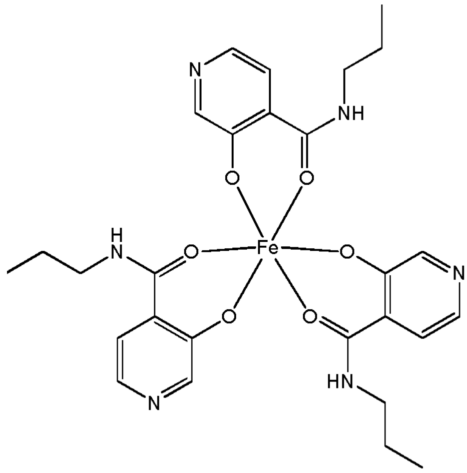 , |
| Exp. 6 | 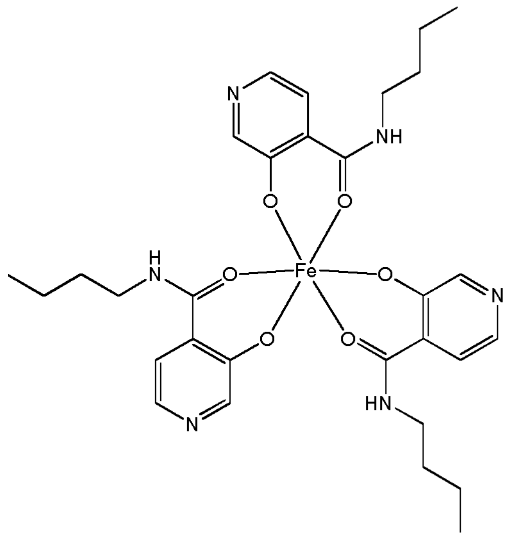 , |
| Exp. 7 | 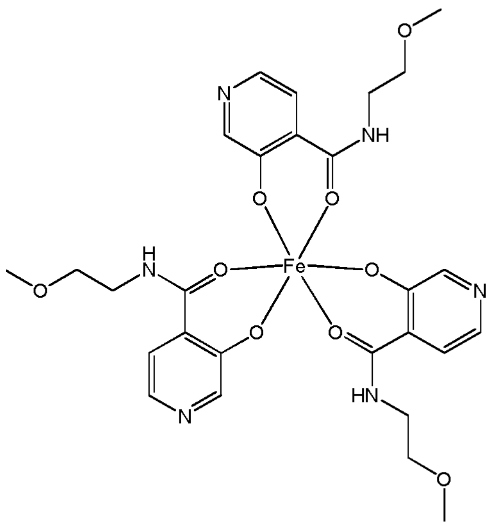 , |

(continued)

| | |
|---|---|
| Exp. 8 | 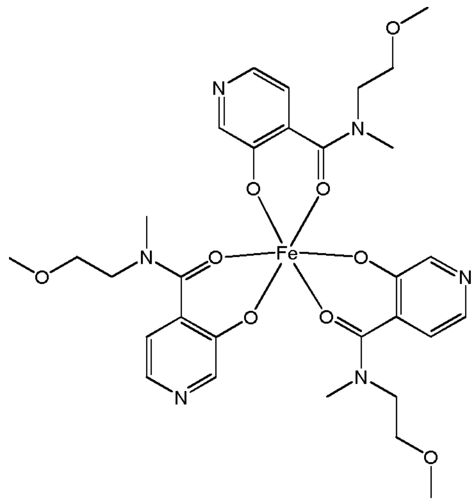 , |
| Exp. 9 | 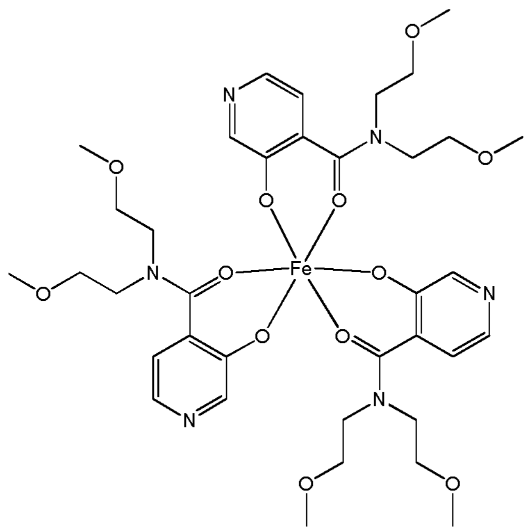 , |
| Exp. 10 | 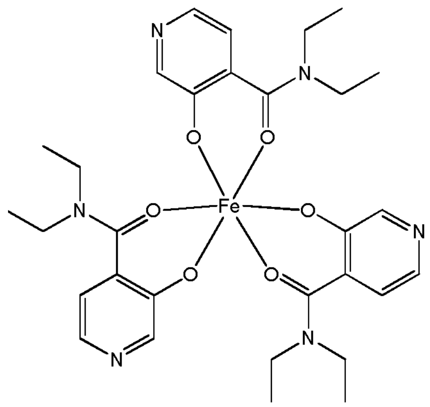 , |

(continued)

| | |
|---|---|
| Exp. 11 | 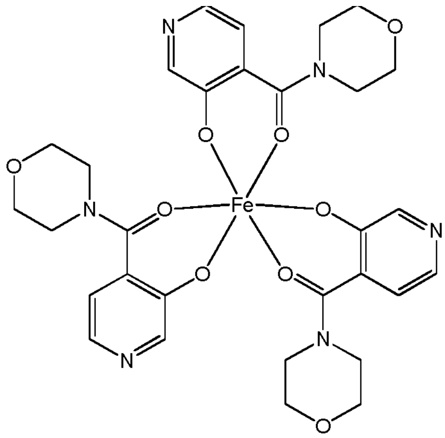 , |
| Exp. 12 | 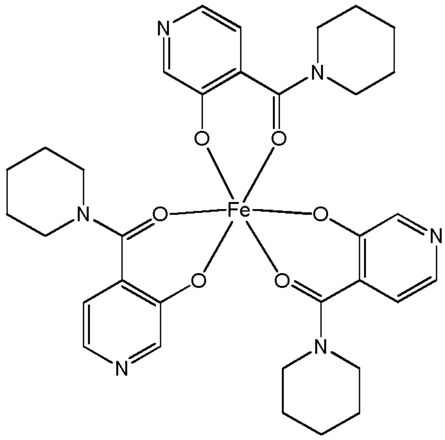 and |
| Exp. 13 | 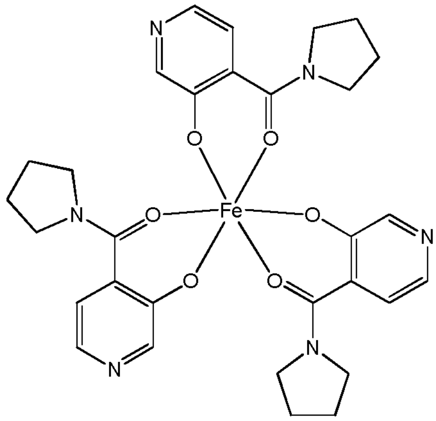 |

**9.** Iron(III) complex compounds for the use according to one of the claims 1 through 8 for the treatment and prophylaxis of iron deficiency symptoms and iron deficiency anemias and the symptoms associated therewith

**10.** Iron(III) complex compounds for the use according to claim 9, wherein the symptoms include: fatigue, listlessness, lack of concentration, low cognitive efficiency, difficulties in finding the right words, forgetfulness, unnatural pallor, irritability, acceleration of heart rate (tachycardia), sore or swollen tongue, enlarged spleen, desire for strange foods (pica), headaches, lack of appetite, increased susceptibility to infections, depressive moods.

**11.** Iron(III) complex compounds for the use according to one of the claims 1 through 10 for the treatment and prophylaxis of iron deficiency anemia in pregnant women, latent iron deficiency anemia in children and adolescents, iron deficiency anemia caused by gastrointestinal abnormalities, iron deficiency anemia due to blood loss, such as gastrointestinal hemorrhage (e.g. due to ulcers, carcinoma, hemorrhoids, inflammatory disorders, taking of acetylsalicylic acid), iron deficiency anemia caused by menstruation, iron deficiency anemia caused by injuries, iron deficiency anemia due to psilosis (sprue), iron deficiency anemia due to reduced dietary iron uptake, in particular in selectively eating children and adolescents, immunodeficiency caused by iron deficiency anemia, brain function impairment caused by iron deficiency anemias, restless leg syndrome caused by iron deficiency anemias, iron deficiency anemias in the case of cancer, iron deficiency anemias caused by chemotherapies, iron deficiency anemias triggered by inflammation (AI), iron deficiency anemias in the case of congestive cardiac insufficiency (CHF; congestive heart failure), iron deficiency anemias in the case of chronic renal insufficiency stage 3-5 (CKD 3-5; chronic kidney diseases stage 3-5), iron deficiency anemias triggered by chronic inflammation (ACD), iron deficiency anemias in the case of rheumatoid arthritis (RA), iron deficiency anemias in the case of systemic lupus erythematosus (SLE) and iron deficiency anemias in the case of inflammatory bowel diseases (IBD).

**12.** Iron(III) complex compounds for the use according to one of the claims 1 through 11 for oral administration.

**13.** Iron(III) complex compounds according to claim 12 for administration in the form of a tablet or a capsule.

**14.** A medicament containing iron(III) complex compounds as defined in one of the claims 1 through 8.

**15.** A medicament containing iron(III) complex compounds as defined in one of the claims 1 through 8 and at least one physiological compatible carrier or excipient.

**16.** Compositions containing iron(III) complex compounds as defined in one of the claims 1 through 8, in combination with at least one further medicament which acts on the iron metabolism.

**Revendications**

**1.** Des composés complexes de 3-hydroxyisonicotinamide de fer (III) ou des sels pharmaceutiquement acceptables de ceux-ci pour l'utilisation dans le traitement et la prophylaxie de la carence en fer et des anémies ferriprives.

**2.** Des composés complexes de fer (III) pour l'utilisation selon la revendication 1, contenant au moins un ligand selon la formule (I) :

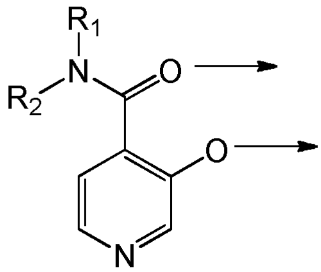

(I)

dans lequel
les flèches représentent chacune une liaison de coordination avec un ou plusieurs atomes de fer différents ;
$R_1$ et $R_2$ sont les mêmes ou différents et sont chacun sélectionnés dans le groupe composé de :

- l'hydrogène et
- de l'alkyle éventuellement substitué, ou dans lequel

$R_1$ et $R_2$ forment, ensemble avec l'atome d'azote, auquel ils sont liés, un cycle à 3 à 6 membres éventuellement substitué, qui peut éventuellement comprendre un autre hétéroatome ;
ou des sels pharmaceutiquemt acceptables de ceux-ci.

**3.** Des composés complexes de fer (III) pour l'utilisation selon la revendication 1 ou la revendication 2, contenant au moins un ligand selon la formule (I) :

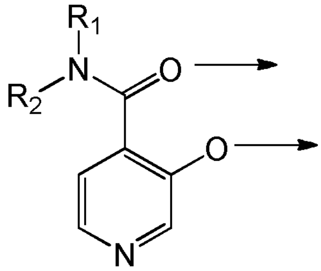

(I)

dans lequel
les flèches représentent chacune une liaison de coordination avec un ou plusieurs atomes de fer différents ;
$R_1$ et $R_2$ sont les mêmes ou différents et sont chacun sélectionnés dans le groupe composé de :

- l'hydrogène et
- de l'alkyle éventuellement substitué, ou dans lequel

$R_1$ et $R_2$ forment, ensemble avec l'atome d'azote, auquel ils sont liés, un cycle à 5 à 6 membres éventuellement substitué, qui peut éventuellement comprendre un autre hétéroatome ;
ou des sels pharmaceutiquemt acceptables de ceux-ci.

**4.** Des composés complexes de fer (III) pour l'utilisation selon l'une des revendications 1 à 3, contenant au moins un ligand selon la formule (I) :

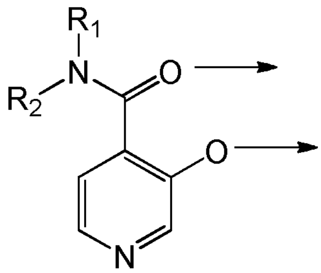

(I)

dans lequel
les flèches représentent chacune une liaison de coordination avec un ou plusieurs atomes de fer différents ;
$R_1$ et $R_2$ sont les mêmes ou différents et sont chacun sélectionnés dans le groupe composé de :

- l'hydrogène et
- de l'alkyle éventuellement substitué par un groupe alcoxy ou hydroxy, de préférence par un groupe alcoxy, comprenant aussi de l'alkyle, dans lequel un ou deux groupes méthylène ($-CH_2-$) peuvent être remplacés par -O-, ou dans lequel

$R_1$ et $R_2$ forment, ensemble avec l'atome d'azote, auquel ils sont liés, un cycle à 5 à 6 membres éventuellement substitué, qui peut éventuellement comprendre un autre hétéroatome ;
ou des sels pharmaceutiquemt acceptables de ceux-ci.

**5.** Des composés complexes de fer (III) pour l'utilisation selon l'une des revendications 1 à 4, contenant au moins un ligand selon la formule (I) :

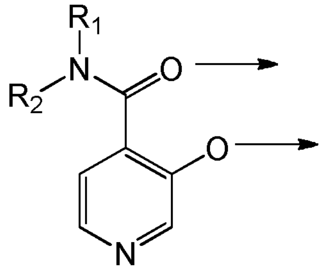

(I)

dans lequel

les flèches représentent chacune une liaison de coordination avec un ou plusieurs atomes de fer différents ;

$R_1$ et $R_2$ sont les mêmes ou différents et sont chacun sélectionnés dans le groupe composé de : l'hydrogène, de méthyle, d'éthyle, de propyle, d'isopropyle, de n-butyle, de sek-butyle, d'isobutyle, de méthoxyéthyle, d'éthoxyéthyle, de méthoxypropyle et d'éthoxypropyle, d'éthoxyéthoxyéthyle de propoxyéthoxyéthyle ; ou dans lequel

$R_1$ et $R_2$ forment, ensemble avec l'atome d'azote, auquel ils sont liés, un cycle hétérocyclique à 5 à 6 membres, qui est sélectionné dans le groupe composé de morpholinyle, de piperidinyle et de pyrrolidinyle ;

ou des sels pharmaceutiquemt acceptables de ceux-ci.

**6.** Des composés complexes de fer (III) pour l'utilisation selon l'une des revendications 1 à 5, contenant au moins un ligand selon la formule (I) :

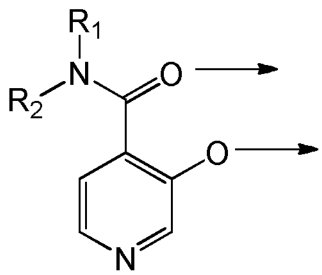

(I)

dans lequel

les flèches représentent chacune une liaison de coordination avec un ou plusieurs atomes de fer différents ;

$R_1$ et $R_2$ sont les mêmes ou différents et sont chacun sélectionnés dans le groupe composé de l'hydrogène, de l'alkyle éventuellement substitué, sélectionné dans le groupe composé de méthyle, d'éthyle, de propyle, de n-butyle et d'alkyle substitué par un groupe méthoxy ou un groupe éthoxy ;

ou dans lequel

$R_1$ et $R_2$ forment, ensemble avec l'atome d'azote, auquel ils sont liés, un cycle hétérocyclique à 5 à 6 membres, qui est sélectionné dans le groupe composé de morpholinyle, de piperidinyle et de pyrrolidinyle ;

ou des sels pharmaceutiquemt acceptables de ceux-ci.

**7.** Des composés complexes de fer (III) pour l'utilisation selon l'une des revendications 1 à 6 et selon la formule (II) :

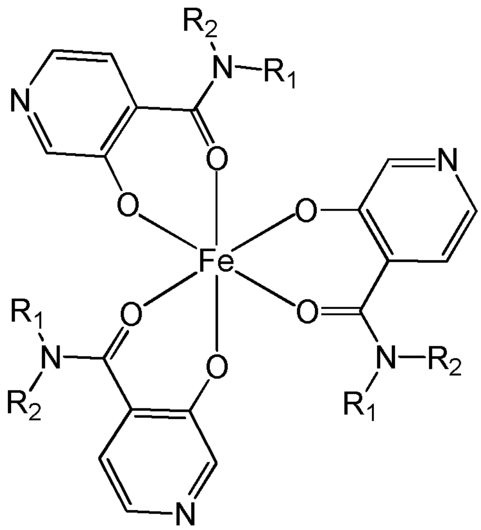

(II)

dans lequel $R_1$ et $R_2$ sont tels que définis dans une des revendications précédentes ou des sels pharmaceutiquemt acceptables de ceux-ci.

8. Des composés complexes de fer (III) pour l'utilisation selon l'une des revendications 1 à 7, lesquels sont sélectionnés dans le groupe :

| | |
|---|---|
| Exemple 1 | 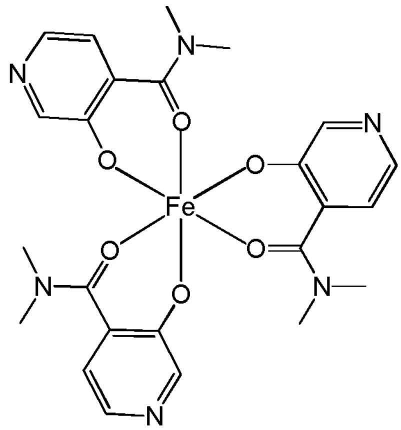 , |
| Exemple 2 | 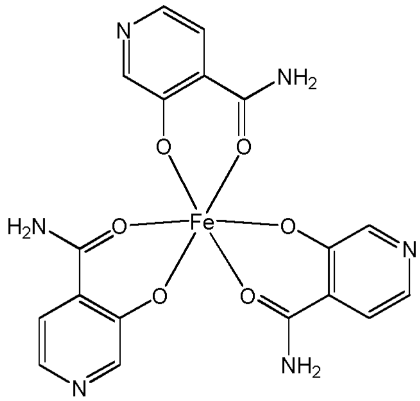 , |

(suite)

| | |
|---|---|
| Exemple 3 | 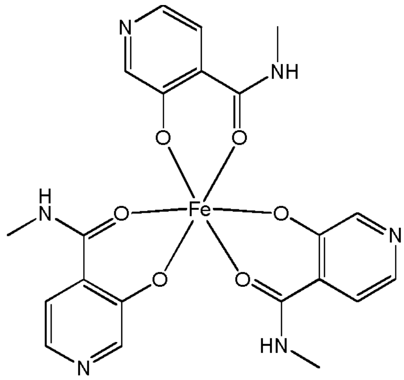 , |
| Exemple 4 | 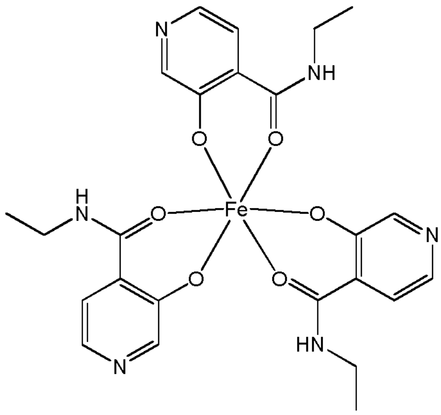 , |
| Exemple 5 | 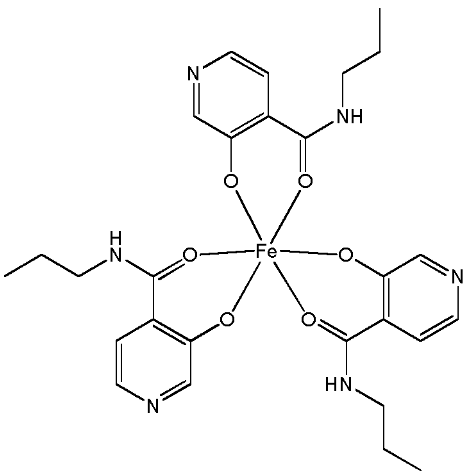 , |

(suite)

| | |
|---|---|
| Exemple 6 | 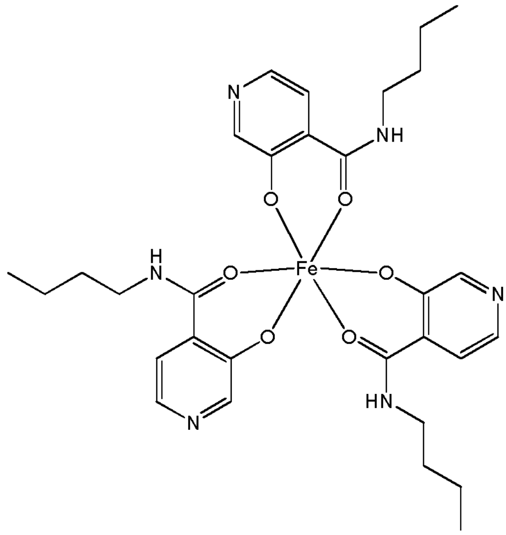 , |
| Exemple 7 | 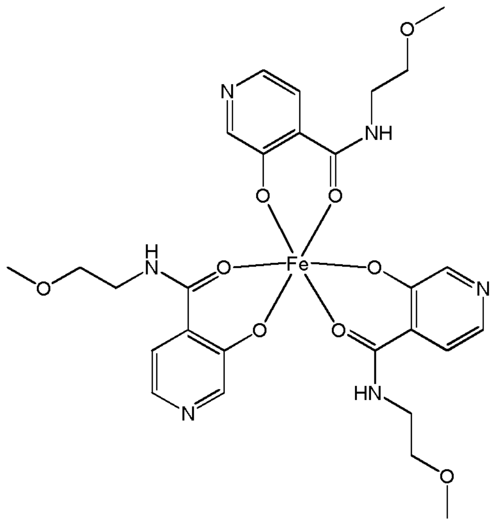 , |
| Exemple 8 | 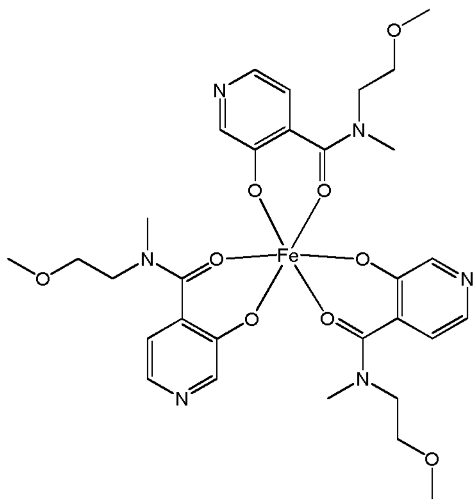 , |

(suite)

| | |
|---|---|
| Exemple 9 | 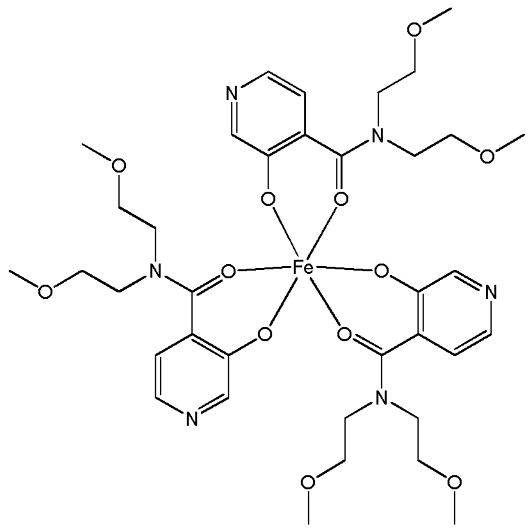 , |
| Exemple 10 | 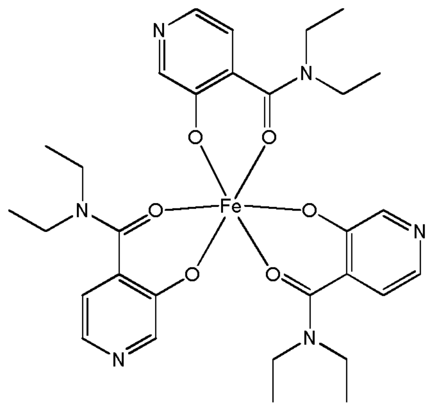 , |
| Exemple 11 | 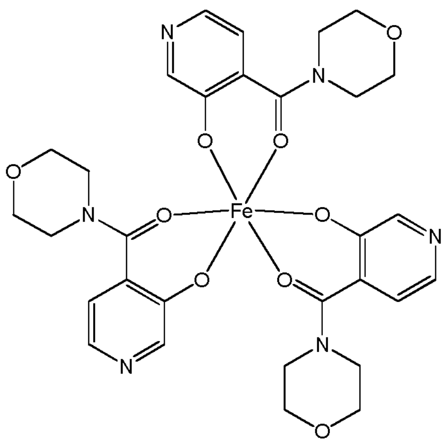 , |

(suite)

| | |
|---|---|
| Exemple 12 | 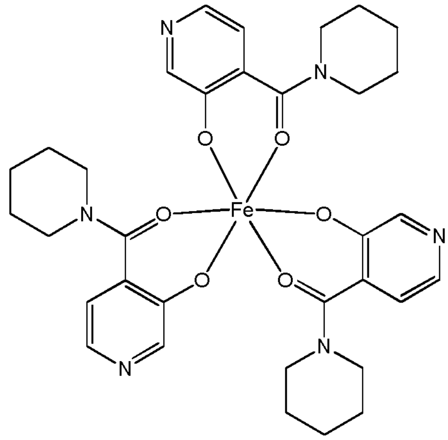 and |
| Exemple 13 | 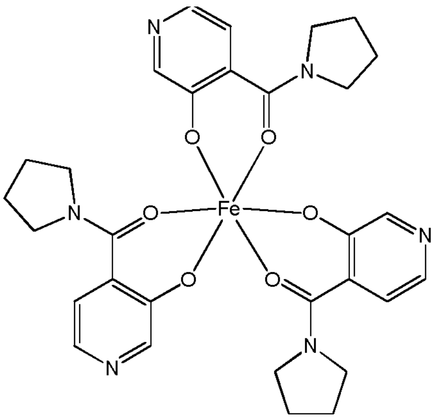 |

**9.** Des composés complexes de fer (III) pour l'utilisation selon l'une des revendications 1 à 8 dans le traitement et la prophylaxie des symptômes de la carence en fer et des anémies ferriprives.

**10.** Des composés complexes de fer (III) pour l'utilisation selon la revendication 9, dans lequel les symptômes comprennent : de la fatigue, de l'apathie, du manque de concentration, de la faible efficacité cognitive, des difficultés de trouver les mots adéquats, de l'oubli, de la pâleur non naturelle, de l'irritabilité, de l'accélération de la fréquence cardiaque (tachycardie), de la langue endolorie ou gonflée, de l'hypertrophie de la rate, du désir pour des aliments étranges (pica), des maux de tête, du manque d'appétit, de la susceptibilité accrue aux infections, des humeurs dépressives.

**11.** Des composés complexes de fer (III) pour l'utilisation selon l'une des revendications 1 à 10 dans le traitement et la prophylaxie de l'anémie ferriprive chez des femmes enceintes, de l'anémie ferriprive latente chez des enfants et des adolescents, de l'anémie ferriprive par suite d'anomalies gastrointestinales, de l'anémie ferriprive par suite de pertes de sang, telles que causées par des saignements gastrointestinaux (par exemple par suite d'ulcères, de carcinomes, d'hémorroïdes, de troubles inflammatoires, de prise de l'acide acétylsalicylique), de l'anémie ferriprive par suite de la menstruation, de l'anémie ferriprive par suite de blessures, de l'anémie ferriprive par suite de psilosis (sprue), de l'anémie ferriprive par suite d'une absorption réduite de fer dans l'alimentation, notamment chez des enfants et des adolescents mangeant sélectivement, de l'immunodéficience causée par l'anémie ferriprive, de l'atteinte de la performance cérébrale causée par l'anémie ferriprive, du syndrome des jambes sans repos causé par l'anémie ferriprive, de l'anémie ferriprive dans le cas de cancer, de l'anémie ferriprive causée par des chimiothérapies, de l'anémie ferriprive causée par l'inflammation (AI), de l'anémie ferriprive dans le cas d'une défaillance cardiaque congestive (CHF ; congestive heart failure), de l'anémie ferriprive dans le cas d'une défaillance rénale chronique stade 3-5 (CKD 3-5 : chronic kidney diseases stage 3-5), de l'anémie ferriprive causée par l'inflammation chronique (ACD), de l'anémie ferriprive dans le cas d'une arthrite rhumatoïde (RA ; rheumatoid arthritis), de l'anémie ferriprive dans le cas d'un lupus érythémateux systémique (SLE ; systemic lupus erythematosus) et de l'anémie ferriprive dans le cas des maladies intestinales inflammatoires (IBD ; inflammatory bowel diseases).

12. Des composés complexes de fer (III) pour l'utilisation selon l'une des revendications 1 à 11 pour l'administration orale.

13. Des composés complexes de fer (III) pour l'utilisation selon la revendication 12 pour l'administration comme comprimé ou comme capsule.

14. Médicament contenant des composés complexes de fer (III) tels que définis dans une des revendications 1 à 8.

15. Médicament contenant des composés complexes de fer (III) tels que définis dans une des revendications 1 à 8 ainsi qu'au moins un support ou un excipient physiologiquement acceptable.

16. Compositions contenant des composés complexes de fer (III) tels que définis dans une des revendications 1 à 8, en combinaison avec au moins un autre médicament, qui agit sur le métabolisme de fer.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 20081455586 A
- WO 2007062546 A
- WO 20040437865 A
- US 2003236224 A
- EP 150085 A
- CN 101481404
- EP 939083 A
- JP 02083400 A
- US 474670 A
- CN 1687089
- US 2009035385 A
- EP 308362 A
- ES 2044777
- FR 19671016
- EP 159194 A
- EP 138420 A
- EP 107458 A
- EP 0120670 A
- WO 2006037449 A
- WO 2012130882 A
- WO 2012163938 A
- WO 2011117225 A


### In der Beschreibung aufgeführte Nicht-Patentliteratur


- **M.W. HENTZE.** Balancing acts: molecular control of mammalian iron metabolism. *Cell,* 2004, vol. 117, 285-297
- *Biometals,* 2009, vol. 22, 701-710
- **CRUM ; FUCHSMAN.** *J. Heterocyclic Chem.,* 1966, vol. 3, 252-256
- **J.D. CRUM ; C.H. FUCHSMAN.** *J. Heterocyclic Chem.,* 1966, vol. 3, 252-256